# FASCICULE DE BREVET EUROPEEN

(11) **EP 2 931 295 B1**
(45) Date de publication et mention de la délivrance du brevet: **19.06.2019**
(21) Numéro de dépôt: 13792678.8
(22) Date de dépôt: 18.11.2013
(51) Int. Cl.: A61K 36/05, A61P 25/28

(54) **EXTRAIT D'ALGUES COMPRENANT DES POLYSACCHARIDES POLYANIONIQUES SULFATÉS ET NON SULFATÉS ET SES APPLICATIONS**
ALGENEXTRAKT MIT SULFATIERTEN UND NICHT-SULFATIERTEN POLYANIONISCHEN POLYSACCHARIDEN UND VERWENDUNG DAVON
ALGAL EXTRACT COMPRISING SULPHATED AND NON-SULPHATED POLYANIONIC POLYSACCHARIDES AND USES THEREOF

(30) Priorité: 11.12.2012 FR 1261909
(43) Date de publication de la demande: 21.10.2015
(73) Titulaire: Amadeite, 56580 Brehan (FR)
(72) Inventeur: DEMAIS, Hervé, F-56700 Merlevenez (FR); LE CHEVILLER, Isabelle., 56580 Bréhan (FR); BALUSSON, Sébastien, Saint Etienne du Gue de L'Isle (FR)
(74) Mandataire: Lavoix
(86) Numéro de dépôt international: PCT/EP2013/074105
(87) Numéro de publication internationale: WO 2014/090515

(56) Documents cités:
- US-A1- 2005 196 410
- MARC LAHAYE ET AL: "Chemical composition and 13C NMR spectroscopic characterisation of ulvans from Ulva (Ulvales, Chlorophyta)", JOURNAL OF APPLIED PHYCOLOGY, vol. 11, no. 1, 1 janvier 1999 (1999-01-01), pages 1-7, XP055068568, ISSN: 0921-8971, DOI: 10.1023/A:1008063600071
- LAHAYE M ET AL: "Structure and function properties of Ulvan, a polysaccharide from green seaweeds", BIOMACROMOLECULES, AMERICAN CHEMICAL SOCIETY, US, vol. 8, no. 6, 1 juin 2007 (2007-06-01), pages 1765-1774, XP002522792, ISSN: 1525-7797, DOI: 10.1021/BM061185Q [extrait le 2007-04-26]
- BIN HUI ET AL: "Sulfated polymannuroguluronate, a novel anti-acquired immune deficiency syndrome drug candidate, decreased vulnerability of PC12 cells to human immunodeficiency virus tat protein through attenuating calcium overload", JOURNAL OF NEUROSCIENCE RESEARCH, vol. 86, no. 5, 1 avril 2008 (2008-04-01), pages 1169-1177, XP055068677, ISSN: 0360-4012, DOI: 10.1002/jnr.21566

## Description

La présente invention concerne un extrait d'algues comprenant des polysaccharides polyanioniques sulfatés et non sulfatés, pour utilisation dans des applications thérapeutiques et prophylactiques.

Les espèces d'ulves (Ulvales, Chlorophyta) sont des algues abondantes trouvées dans la zone intertidale ou estran. Elles colonisent les substrats durs, ancrés par un disque de fixation, mais certaines espèces peuvent également donner lieu à des formes vivantes libres, à la dérive. Les ulves sont des algues à croissance rapide et opportunistes quant à l'espace et l'absorption des nutriments. Leur croissance dans la colonne d'eau est particulièrement observée dans les eaux côtières eutrophisées et dans les lagunes où *Ulva* sp. prolifère sous la forme de "marées vertes" (Fletcher, 1996). Il en résulte souvent une production de masse et des échouages massifs produisant des gaz nocifs lors de leur accumulation (Morand et Briand, 1996). Jusqu'à présent, cette biomasse a possédé une très faible valeur ajoutée et les moyens de l'utiliser en dehors du composte (Mazé et al. 1993, Cuomo et al. 1995), de la production de méthane (Briand et Morand, 1997), de la consommation alimentaire humaine (Pérez, 1997) ou comme base de papier (Nicolucci et Monegato 1993) pourraient permettre de profiter de leurs propriétés spécifiques.

A l'heure actuelle, l'utilisation des algues vertes est essentiellement basée sur des usages permettant d'ouvrir de nouvelles voies d'élimination pour ces algues considérées indésirables. Les voies d'application envisagées concernent la production de biogaz (Briand & Morand 1997, Morand & Briand 1999, Morand et al., 2006), les biomatériaux (bioplastiques) (Chiellini et al., 2008), godets biodégradables (Sassi et al., 2008), papiers et cartons d'emballage (Nicolucci & Monegata, 1993), nano-composites de charge (Demais et al., 2006a, 2006b), la nutrition animale: aliments détoxifiants pour animaux (Demais et al.,2006a), protéines et facteurs d'appétence pour élevage de poissons (DeBose et al., 2008, Kut Guroy et al., 2007) et la cosmétique (tensioactifs) (Ranson et al., 2008).

Les propriétés chimiques et physico-chimiques des polysaccharides contenus dans ces algues en font des candidats attrayants comme nouveaux polymères fonctionnels et biologiquement actifs dans les domaines de la consommation humaine et animale, pharmaceutique, chimique, de l'aquaculture et agricoles (Robic A, et al. Carbohydrate Polymers 77 (2009) 206-216; Lahaye M Robic A, Biomacromolecules Vol 8 N° 6 , 2007).

Selon un des ces aspects, la présente invention concerne un extrait d'algues de l'ordre des ulvales, en particulier un extrait d'algues vertes de type *Ulva,* comprenant des polysaccharides polyanioniques sulfatés et non sulfatés dont la taille est inférieure ou égale à 50 kDa. pour utilisation selon la revendication 1.

En particulier, lesdits polysaccharides de l'extrait d'algues comprennent du mannose et/ou de l'arabinose. Plus particulièrement, lesdits polysaccharides comprennent au moins 0.005% de mannose et/ou au moins 0.005% d'arabinose, en poids par rapport au poids de la matière sèche totale de l'extrait d'algues, notamment au moins 0.01 % de mannose et/ou au moins 0.01 % d'arabinose. Encore plus particulièrement, lesdits polysaccharides comprennent du mannose en une quantité allant de 0.01 à 0.20% et de l'arabinose en une quantité allant de 0.01 à 0.5%, en poids par rapport au poids de la matière sèche totale de l'extrait d'algues, notamment du mannose en une quantité allant de 0.03 à 0.15% et de l'arabinose en une quantité allant de 0.01 à 0.2%.

De façon particulière, lesdits polysaccharides comprennent en outre:
- du galactose;
- du glucose;
- du rhamnose;
- du xylose; et
- de l'acide glucuronique.

Plus particulièrement encore, lesdits polysaccharides comprennent :
- de 0.05 à 0.5% de galactose, notamment de 0.1 à 0.4%;
- de 0.005 à 0.05% de glucose, notamment de 0.01 à 0.03%;
- de 2 à 15 % de rhamnose, notamment de 5 à 10%;
- de 0.1 à 1% de xylose, notamment de 0.3 à 0.7%; et
- de 1 à 7% d'acide glucuronique, notamment de 1 à 5%;
en poids par rapport au poids de la matière sèche totale de l'extrait d'algues.

Ainsi, on peut par exemple citer un extrait d'algues selon l'invention comprenant :
- du mannose ;
- de l'arabinose ;
- du galactose;
- du glucose;
- du rhamnose;
- du xylose; et
- de l'acide glucuronique.

On peut plus particulièrement citer par exemple un extrait d'algues selon l'invention comprenant:
- de 0.01 à 0.20% de mannose, notamment de 0.03 à 0.15% ;
- de 0.01 à 0.5% d'arabinose, notamment de 0.01 à 0.2% ;
- de 0.05 à 0.5% de galactose, notamment de 0.1 à 0.4%;
- de 0.005 à 0.05% de glucose, notamment de 0.01 à 0.03%;
- de 2 à 15 % de rhamnose, notamment de 5 à 10%;
- de 0.1 à 1% de xylose, notamment de 0.3 à 0.7%; et
- de 1 à 7% d'acide glucuronique, notamment de 1 à 5%;
en poids par rapport au poids de la matière sèche totale de l'extrait d'algues.

On peut encore plus particulièrement citer par exemple un extrait d'algues selon l'invention comprenant :
- 0.09% de mannose;
- 0.1% d'arabinose ;
- 0.3% de galactose;
- 0.02% de glucose ;
- 8.1% de rhamnose;
- 0.5% de xylose; et
- 2.6% d'acide glucuronique;
en poids par rapport au poids de la matière sèche totale de l'extrait d'algues.

Plus particulièrement, l'extrait d'algues comprend des polysaccharides polyanioniques sulfatés et non sulfatés dont la taille est inférieure à 40, 30, 20 ou 15 kDa. Plus particulièrement encore, les polysaccharides polyanioniques sulfatés et non sulfatés de l'extrait d'algues selon l'invention ont une taille inférieure ou égale à 15 kDa.

Un dalton (Da) est une unité massique définie comme étant égal à un douzième de la masse d'un atome de carbone 12, masse qui s'avérera ensuite estimée à partir d'un mélange de plusieurs isotopes (principalement carbone 12 et carbone 13, possédant respectivement 6 et 7 neutrons en plus des 6 protons de tout atome de carbone). Un dalton est, avec une assez bonne précision, la masse d'un atome d'hydrogène, la valeur exacte étant 1,00794 uma (unité de masse atomique). Le kilodalton (kDa) est égal à 1000 Da.

Dans le cadre de la présente invention, les masses mentionnées en kDa sont déterminées par toute méthode usuellement employée par l'Homme du métier, en particulier les masses des polysaccharides polyanioniques sulfatés et non sulfatés des extraits d'algues selon la présente invention pourront être discriminées par ultrafiltration sur des membranes laissant uniquement filtrer des molécules de tailles prédéterminées.

Par « algues vertes de type *Ulva* » on entend les algues vertes regroupées dans le genre *Ulva,* de la famille des Ulvaceae, de l'ordre des ulvales. On peut notamment citer les espèces et sous-espèces suivantes : *Ulva acanthophora, Ulva anandii, Ulva angusta, Ulva arasakii, Ulva armoricana, Ulva atroviridis, Ulva attenuata, Ulva beytensis, Ulva bifrons, Ulva brevistipitata, Ulva bulbosa, Ulva burmanica, Ulva byssoides, Ulva californica, Ulva chaetomorphoides, Ulva clathrata, Ulva coccinea, Ulva compressa, Ulva conglobata, Ulva cornucopiae, Ulva cornuta, Ulva covelongensis, Ulva crassa, Ulva crassimembrana, Ulva curvata, Ulva dactylifera, Ulva denticulata, Ulva elegans, Ulva elminthoides, Ulva enteromorpha, Ulva erecta, Ulva expansa, Ulva fasciata, Ulva fenestrata, Ulva flexuosa, Ulva gelatinosa, Ulva geminoidea, Ulva gigantea, Ulva grandis, Ulva hendayensis, Ulva hookeriana, Ulva hopkirkii, Ulva indica, Ulva intestinalis, Ulva intestinaloides, Ulva intricata, Ulva intybacea, Ulva javanica, Ulva kylinii, Ulva lactuca, Ulva lactucaefolia, Ulva laetevirens, Ulva laingii, Ulva linearis, Ulva lingulata, Ulva linkiana, Ulva linza, Ulva lippii, Ulva litoralis, Ulva littorea, Ulva lobata, Ulva lubrica, Ulva marginata, Ulva micrococca, Ulva myriotrema, Ulva neapolitana, Ulva nematoidea, Ulva ohnoi, Ulva olivacea, Ulva olivaceum, Ulva pacifica, Ulva papenfussii, Ulva paradoxa, Ulva parva, Ulva parvula, Ulva patengensis, Ulva percursa, Ulva pertusa, Ulva phyllosa, Ulva popenguinensis, Ulva porrifolia, Ulva procera, Ulva profunda, Ulva prolifera, Ulva pseudocurvata, Ulva pseudolinza, Ulva pulchra, Ulva purpurascens, Ulva quilonensis, Ulva radiata, Ulva ralfsii, Ulva ranunculata, Ulva reticulata, Ulva rhacodes, Ulva rigida, Ulva rotundata, Ulva rubens, Ulva saifullahii, Ulva scagelii, Ulva scandinavica, Ulva sericea, Ulva serrata, Ulva simplex, Ulva sorensenii, Ulva spinulosa, Ulva stenophylla, Ulva stipitata, Ulva sublittoralis, Ulva subulata, Ulva taeniata, Ulva tenera, Ulva tetragona, Ulva torta, Ulva tuberosa, Ulva umbilicata, Ulva uncialis, Ulva uncinata, Ulva usneoides, Ulva utricularis, Ulva utriculosa, Ulva uvoides, Ulva ventricosa.*

Plus particulièrement, l'extrait d'algues est un extrait d'algues de l'espèce *Ulva armoricana.*

Selon un mode de réalisation, l'extrait d'algues comprend :
- de 10 à 50 % de carbone ;
- de 1 à 10% d'hydrogène;
- de 1 à 5 % d'azote ;
- de 20 à 50 % d'oxygène ; et
- de 1 à 15 % de soufre ;
en pourcentage massique de la matière sèche totale de l'extrait d'algues.

Plus particulièrement encore, l'extrait d'algues comprend :
- de 15 à 30 % de carbone ;
- de 3 à 6% d'hydrogène ;
- de 1 à 3 % d'azote ;
- de 25 à 40 % d'oxygène ; et
- de 2,5 à 10 % de soufre ;
en pourcentage massique de la matière sèche totale de l'extrait d'algues.

Les autres éléments chimiques présents dans la matière sèche de l'extrait sont notamment représentés par les minéraux (Ca, K, Na, Mg, Al, Cl, l, P, Fe, etc).

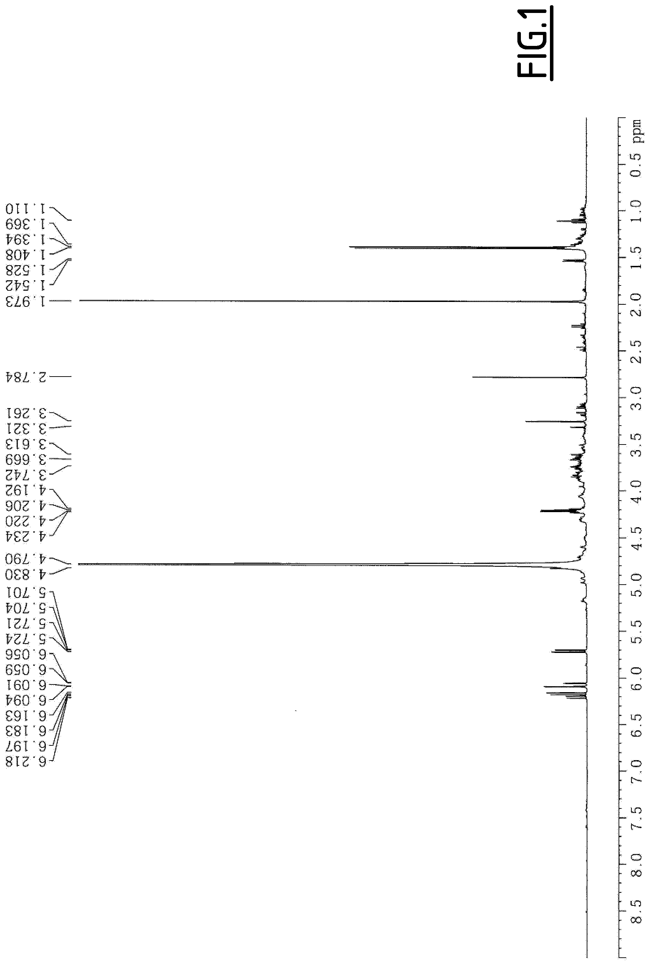

Plus particulièrement, l'extrait d'algues selon l'invention est caractérisé par le spectre RMN ¹H présenté en Figure 1.

Ce spectre RMN ¹H a été enregistré à 298 K sur un spectromètre Bruker Avance 500 équipé d'une sonde cryogénique inverse 5 mm ¹H/¹³C/¹⁵N TCl. Avant l'analyse, les échantillons ont été dissous dans 99,97% d'atome de D₂O. Les déplacements chimiques sont exprimés en ppm par rapport à un étalon externe (acide triméthylsilylpropionique). Aucune suppression du signal HOD n'a été réalisée.

Selon un autre des ces aspects, la présente invention concerne un extrait d'algues de l'ordre des ulvales pour utilisation selon la revendication 12 susceptible d'être obtenu par un procédé de production d'un extrait d'algues de l'ordre des ulvales, en particulier un extrait d'algues vertes de type *Ulva,* dans lequel:
a) les algues sont lavées et dessablées ;
b) lesdites algues sont broyées ;
c) la phase solide du broyat est séparée de sa phase liquide ;
d) ladite phase liquide est clarifiée ;
e) le jus obtenu à l'étape d) est ultrafiltré ; et
f) le jus de filtration obtenu à l'étape e) est concentré puis séché.

Selon un mode de réalisation du procédé les algues sont lavées à l'eau douce.

Elles peuvent être dessablées par tout moyen mis à disposition de l'Homme du métier.

Lesdites algues sont ensuite broyées, notamment au moyen d'un broyeur, comme par exemple un affineur ou un cutteur.

Par la suite, la phase solide du broyat, le marc, est séparée de sa phase liquide, le jus, par pressage du broyat, par exemple à l'aide d'une presse à bande ou à plateaux, ou par centrifugation.

Par « jus », on entend le jus cytoplasmique qui inclue la structure pariétale de la double structure des cellules des algues.

La phase liquide obtenue est ensuite clarifiée, par exemple avec un clarificateur à assiettes, ou par centrifugation, décantation ou filtration (par exemple à poche ou à plaque).

Le jus obtenu est ensuite ultrafiltré.

Selon la réalisation du procédé l'ultrafiltration est réalisée sur une membrane de moins de 50 kDa, notamment sur une membrane de 40, 30, 20 ou 15 kDa. Plus particulièrement, la membrane sera une membrane de 15 kDa.

Cette membrane peut être par exemple une membrane de céramique ou une membrane organique. Plus particulièrement, la membrane sera une membrane de céramique.

Le jus de filtration obtenu peut ensuite être concentré, par exemple par osmose inverse, évaporation ou précipitation, puis séché par exemple par lyophilisation ou atomisation.

Optionnellement, l'extrait obtenu pourra ensuite être broyé de nouveau, afin d'obtenir une poudre homogène en terme de granulométrie.

Selon un de ces aspects, le procédé se déroule en partie à température ambiante. Par température ambiante, on entend une température comprise entre 5 et 25°C.

Selon un autre de ces aspects, le procédé se déroule en partie à une température comprise entre 4 et 10°C, ceci afin d'éviter les développements microbiens.

Ce procédé diffère de la plupart des procédés décrits dans l'art antérieur du fait de l'absence d'une étape impliquant une précipitation de l'extrait d'algues. Il se distingue également des précédents procédés d'extraction de par l'absence d'utilisation de solvants, en particulier organiques, ce qui représente un atout majeur du point de vue écologique.

Selon un de ces aspects, la présente invention concerne également une composition pharmaceutique pour utilisation selon la revendication 17 comprenant un extrait d'algues tel que précédemment décrit ainsi qu'au moins un excipient pharmaceutiquement acceptable.

La présente invention concerne un extrait d'algues tel que précédemment décrit pour son utilisation dans la prévention et/ou le traitement de désordres neurologiques, de la dépression, de l'anxiété, dans la prévention et/ou le traitement de la maladie d'Alzheimer, dans la prévention et/ou le traitement des douleurs chroniques neuropathiques et inflammatoires, de la douleur cutanée (de la peau, des tissus sous-cutanés et organes associés) ou somatique.

Par « désordres neurologiques », on entend les maladies du système nerveux, en particulier du cerveau. Parmi ces maladies, on peut notamment citer les pathologies du cerveau comme la maladie de Parkinson, la sclérose en plaques, la maladie d'Alzheimer, les démences, les migraines, les céphalées.

Par « dépression », on entend un trouble de l'humeur caractérisé essentiellement par un état de perte de motivation ou d'élan vital chez un individu, associé ou non à différents symptômes. Les symptômes les plus caractéristiques sont une perte d'espoir, d'envie, d'estime de soi. D'autres signes peuvent survenir, tels que la fatigue, la tristesse, des pensées négatives, des idées noires, des intentions suicidaires, de l'anxiété ou de l'angoisse et dans certains rares cas extrêmes, des hallucinations.

On retrouve le terme de dépression sous les appellations « trouble dépressif récurrent », « dépression nerveuse », « dépression clinique », « dépression unipolaire », «épisode dépressif majeur et caractérisé » ou encore «syndrome dépressif ». L'appellation « les dépressions » désigne l'ensemble des types de dépression. Les termes « dépressivité » ou « sentiment dépressif » sont également utilisés. Le langage courant évoque également la « déprime », qui présente des symptômes similaires, mais plus atténués.

Par « anxiété », on entend un état psychologique et physiologique caractérisé par des composants somatiques, émotionnels, cognitifs et comportementaux. En l'absence ou en présence de stress psychologique, l'anxiété peut créer des sentiments de peur, d'inquiétude, de difficulté et de crainte. L'anxiété est considérée comme une réaction normale dans une situation stressante. Lorsque l'anxiété devient excessive, elle peut être classifiée sous la dénomination de « trouble de l'anxiété».

Ainsi un extrait d'algues peut être utilisé aussi bien dans des applications vétérinaires, comme par exemple pour prévenir et/ou traiter le stress chez les animaux d'élevage et de compagnie ou comme agent anti-dépresseur pour animaux d'élevage ou animaux de compagnie, que dans des applications destinées à l'être humain, par exemple par le biais de compléments alimentaires à visée santé sans effets secondaires ou d'un médicament, pour par exemple prévenir et/ou traiter les désordres liés au stress, à l'anxiété, à la dépression, ainsi que comme agent permettant d'améliorer la mémoire, notamment chez des étudiants, pour lutter contre le vieillissement ou encore dans le cadre du traitement de la maladie d'Alzheimer.

Les excipients pharmaceutiquement acceptables sont choisis selon la forme pharmaceutique et le mode d'administration souhaité, parmi les excipients habituels qui sont connus de l'Homme du métier.

Dans les compositions pharmaceutiques pour l'administration orale, sublinguale, sous-cutanée, intramusculaire, intra-veineuse, topique, locale, intratrachéale, intranasale, transdermique ou rectale, l'extrait d'algues tel que défini ci-dessus, peut être administré sous forme de doses unitaires, en mélange avec des excipients pharmaceutiques classiques, aux animaux et aux êtres humains, pour la prévention ou le traitement des désordres précités.

Les formes d'administration appropriées comprennent les formes par voie orale telles que les comprimés, les gélules molles ou dures, les poudres, les granules et les solutions ou suspensions orales, les formes d'administration sublinguale, buccale, intratrachéale, intraoculaire, intranasale, par inhalation, les formes d'administration topique, parentérale telle que transdermique, sous cutanée, intramusculaire ou intraveineuse, les formes d'administration rectale et les implants. Pour l'application topique, on peut utiliser les composés selon l'invention dans des crèmes, gels, pommades ou lotions.

Lorsqu'une composition solide sous forme de comprimés est préparée, l'ingrédient actif principal peut être mélangé avec un véhicule pharmaceutique, tel que la gélatine, l'amidon, le lactose, le stéarate de magnésium, le talc, la gomme arabique ou analogues.

Les comprimés peuvent également être enrobés avec du saccharose, un dérivé cellulosique, ou d'autres matières appropriées ou encore ils peuvent être traités de telle sorte qu'ils aient une activité prolongée ou retardée et qu'ils libèrent d'une façon continue une quantité prédéterminée de principe actif.

Une préparation sous forme de gélules peut par exemple être obtenue en mélangeant l'ingrédient actif avec un diluant et en versant le mélange obtenu dans des gélules molles ou dures.

Les compositions pharmaceutiques contenant un extrait d'algues conformément à l'invention, peuvent aussi se présenter sous forme liquide, par exemple, sous forme de solutions, d'émulsions, de suspensions ou de sirops, et notamment sous une forme adaptée pour une administration orale ou intranasale, par exemple. Les supports liquides appropriés peuvent être, par exemple, de l'eau, des solvants organiques tels que le glycérol ou les glycols, de même que leurs mélanges, dans des proportions variées, dans l'eau.

Une préparation sous forme de sirop ou d'élixir ou pour l'administration sous forme de gouttes peut également contenir l'ingrédient actif conjointement avec un édulcorant, acalorique par exemple, du méthylparabène et du propylparabène comme antiseptique, ainsi qu'un agent donnant du goût et un colorant approprié.

Les poudres ou les granules dispersibles dans l'eau peuvent par exemple contenir l'ingrédient actif en mélange avec des agents de dispersion ou des agents mouillants, ou des agents de mise en suspension, comme la polyvinylpyrrolidone, de même qu'avec des édulcorants ou des correcteurs de goût.

La dose administrée par jour peut atteindre chez l'homme de 0.5 à 25 mg/kg, en une ou plusieurs prises, en particulier de 1.5 à 18 mg/kg, et encore plus particulièrement de 3 à 15 mg/kg. La dose administrée par jour peut atteindre chez l'animal de 5 à 150 mg/kg, en une ou plusieurs prises, en particulier de 10 à 100 mg/kg et encore plus particulièrement de 20 à 80 mg/kg. En général, la dose journalière de l'extrait d'algues selon l'invention sera la dose efficace la plus faible de l'extrait d'algues capable de produire un effet thérapeutique.

Par « dose efficace », on désigne toute quantité d'une composition qui améliore un ou plusieurs des paramètres caractéristiques de la maladie ou du désordre à traiter.

Il peut y avoir des cas particuliers où des dosages plus élevés ou plus faibles sont appropriés; de tels dosages ne sortent pas du cadre de la présente invention. Selon la pratique habituelle, le dosage approprié à chaque patient, humain ou animal, est déterminé par le médecin ou le vétérinaire selon le mode d'administration, le poids et la réponse dudit patient.

Selon un autre de ses aspects, un extrait d'algues peut être utilisé pour la prévention et/ou le traitement des pathologies précitées dans une composition alimentaire.

Par « composition alimentaire », on entend par exemple tout type d'alicament, de produits alimentaires sous la forme de yaourt ou breuvage, lacté notamment, tout type de matière première, additif ou auxiliaire technologique, sous forme de prémélanges, médicamenteux ou non, destinés à être incorporés dans des aliments, tout type d'aliments complets ou complémentaires, destinés à l'homme ou l'animal.

La présente invention sera illustrée plus en détail par les figures et exemples ci-dessous qui n'en limitent pas la portée.

### FIGURES

**Figure 1** : Spectre RMN ¹H d'un extrait d'algues (EA) selon la présente invention
**Figure 2** : Chromatogramme obtenu avec l'extrait d'algues selon l'invention séparé sur deux colonnes shodex 802 et 803
**Figure 3** **:** Chromatogramme obtenu après l'analyse de dérivés triméthylsilylés de l'échantillon de l'extrait d'algues selon l'invention par chromatographie en phase gazeuse. Avec Ara: Arabinose; Gal: Galactose; Glc: Glucose ; Xyl: Xylose; Man: Mannose; Rha: Rhamnose, GlcA: Acide Glucuronique
**Figure 4** **:** Effets de l'EA sur le nombre d'entrées dans les bras ouverts dans le test du labyrinthe en croix surélevé
**Figure 5** **:** Effets de l'EA sur le temps passé dans les bras ouverts dans le test du labyrinthe en croix surélevé
**Figure 6** : Effets de l'EA sur le nombre total d'appuis cumulés sur les deux leviers dans le test d'apprentissage de l'évitement d'un stimulus lumineux aversif
**Figure 7** : Effets de l'EA sur le nombre total d'appuis sur les leviers actif (LA) et inactif (LI) à 5 minutes dans le test d'apprentissage du TESLA
**Figure 8** : Effets de l'EA sur le nombre total d'appuis sur les leviers actif (LA) et inactif (LI) à 10 minutes dans le test d'apprentissage du TESLA
**Figure 9** : Effets de l'EA sur le nombre total d'appuis sur les leviers actif (LA) et inactif (LI) à 15 minutes dans le test d'apprentissage du TESLA
**Figure 10** : Effets de l'EA sur le nombre total d'appuis sur les leviers actif (LA) et inactif (LI) à 20 minutes dans le test d'apprentissage du TESLA
**Figure 11** : Effets de l'EA sur la durée d'immobilité(s) dans le prétest du désespoir comportemental à J14 (s, Moyenne ± ESM)
**Figure 12** : Effets de l'EA sur la durée d'immobilité(s) dans le test de désespoir comportemental à J15 (s, Moyenne ± ESM)

### EXEMPLES

### Exemple 1: Préparation d'un extrait d'algues

Une tonne d'algues vertes de type *Ulva,* fraîches, brutes, sont lavées à l'eau douce et dessablées à l'aide d'une machine à laver les algues.

Sauf indications contraires, les étapes du procédé sont réalisées à température ambiante.

Les algues (1 tonne d'algues égouttées à 8% de matière sèche) sont ensuite broyées en fines particules au moyen d'un affineur industriel (marque Inotec type "I175CDI-75D"). Par « fines particules », on entend des particules dont la taille est comprise entre 50 et 1000 nm, avec deux populations, la première dont les tailles sont comprises entre 50 et 200 nm, la seconde dont les tailles sont comprises entre 600 et 1000 nm.

Le broyat est ensuite pressé au moyen d'une presse à bande industrielle de marque Flottweg type "B FRU 800 HK" à un débit d'environ 1 tonne/heure.

Cette étape permet la séparation de la phase solide (marc) de la phase liquide (jus). Le rendement en jus obtenu est de 75%.

Les 750 kg de jus brut obtenus sont ensuite clarifiés au moyen d'un clarificateur à assiettes de marque Flottweg type "AC 2000".

On obtient ainsi 710 kg d'un jus clair à 3.10 % de masse sèche (95 à 98% de rendement en masse) et une crème (2 à 5% en masse).

Par la suite, le jus clair est ultrafiltré sur une membrane céramique (Tami Industries) de 15 kDa.

On obtient ainsi un perméat et un rétentat. Le perméat est conservé jusqu'à l'obtention de 640 kg de jus de filtration (91% de rendement en volume) à 2.2% de matière sèche.

Le jus de filtration (perméat) est alors séché par lyophilisation après concentration par évaporation.

La concentration est réalisée sur un évaporateur simple effet (EVA 1000, Pignat) avec les paramètres suivants: recirculation forcée, débit d'alimentation 10L/h, pression vapeur de 1 bar, pression de vide de 0,3 bar et température d'évaporation de 90°C.

Une première concentration est réalisée avec un débit d'eau évaporée de 8 L/h et le °brix monte de 5.5 (égal à une concentration de matière sèche de 4.5%) à 14.7.

Cette solution est ensuite concentrée une deuxième fois avec un débit d'eau évaporée de 5-6 L/h et le °brix monte jusqu'à 34. La concentration de matière sèche de la solution est déterminée à 38.4%.

La lyophilisation est ensuite réalisée à l'aide d'un appareil Bioblock scientific (modèle CHRIST alpha 1-4 LSC) à une température de congélation de -80C qui est également la température minimale au cours de cette étape.

La poudre obtenue est ensuite broyée avec un broyeur planétaire MiniMill de marque Philips. Le produit a été introduit dans des bols de broyage (10 g de produit dans chaque bol de broyage avec 4 billes en zircone). L'ensemble a été mis en rotation pendant 15 minutes à la vitesse 10.

On obtient ainsi 14 kg de poudre d'extrait d'algues.

### Exemple 2 : Détermination de la taille des polysaccharides polyanioniques sulfatés et non sulfatés d'un extrait d'algues par GPC (Gel Permeation Chromatography)

L'extrait d'algues préparé suivant l'exemple 1 est ultrafiltré sur une membrane de 1000 Da et est dissous à une concentration de 0.5 g/L dans de l'eau. Il est ensuite injecté sur deux colonnes shodex 802 et 803 placées en série (domaine de fractionnement de la colonne 802 : 4.10³ Da et de la colonne 803: 1.7.10⁵ Da). L'éluant utilisé est le nitrate de sodium 0.1 M avec de l'azoture de sodium 0.2% à un débit de 0.5 ml/min. La détection est réalisée via un réfractométre Wyatt et un détecteur à diffusion de la lumière 18 angles Wyatt. Les dn/dc sont pris égaux à 0.150 mL/g.

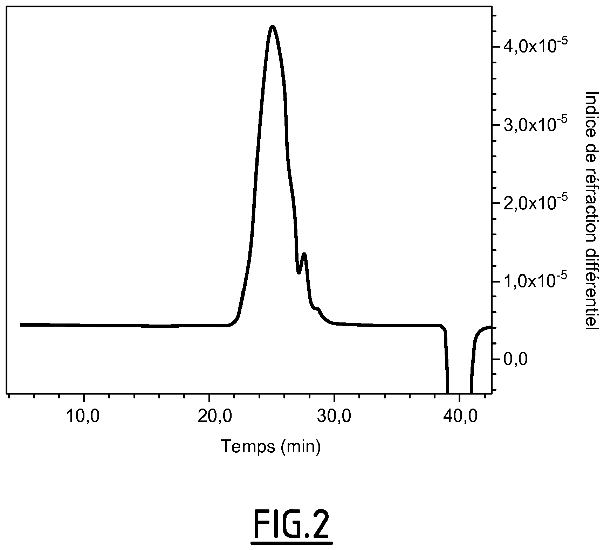

Le chromatogramme détecté par le refractomètre est présenté en figure 2.

On obtient une taille moyenne des polysaccharides d'un extrait d'algues selon l'invention de 4.4 KDa.

### Exemple 3: Détermination de la composition d'un extrait d'algues

L'extrait d'algues préparé selon l'exemple 1 est purifié par ultrafiltration frontale dans des cellules amicon fonctionnant sous agitation. Une membrane en cellulose régénérée de seuil de coupure de 1000 Da est utilisée. 572.1 mg d'échantillon de l'extrait d'algues sont dissous dans 150 ml d'eau ultrapure milli-Q. Cinq litres d'eau sont utilisés pour éliminer toutes les molécules de masse inférieure à 1000 Da. Le rétentat est lyophilisé. 117 mg d'échantillon sont pesés. Le rendement de l'ultrafiltration est donc de 20.5% (p/p). Les analyses suivantes ont été effectuées sur les échantillons ultrafiltrés.

Le ratio des monosaccharides constitutifs des polysaccharides de l'extrait d'algues est déterminé selon la méthode de Kamerling (Kamerling *et al.,* 1975) modifiée par Montreuil (Montreuil *et al.,* 1986). L'identification et le dosage des monosaccharides nécessitent une hydrolyse par méthanolyse du polymère, de façon à n'obtenir que des monomères. Les résidus glycosidiques sont ensuite triméthylsilylés afin de les rendre volatils. Ils sont ainsi identifiés et dosés par chromatographie en phase gazeuse sous forme de méthylglycosides Otriméthylsilylés.

Les réactifs suivants sont utilisés :
- Solution de méthanol/HCI 3N (Supelco) ;
- Carbonate d'argent;
- Myo-inositol ;
- Pyridine ;
- Réactif Sylon BFT (BSTFA +TMCS 99 :1) (Supelco) ; et
- Dichlorométhane.

Le mode opératoire est le suivant : 400 µg de l'extrait d'algues préparé tel que mentionné ci-dessus et 50 µg de myo-inositol sont placés dans un bain à sec en présence de 500 µl d'un mélange méthanol/acide chlorhydrique 3 N (Supelco) pendant 4 heures à 100°C. Après refroidissement à température ambiante, le méthanolysat est neutralisé à l'aide de carbonate d'argent. Les échantillons sont centrifugés 15 minutes à 3000 tr/min et le surnageant est évaporé sous jet d'azote. Les composés sont ensuite dissous dans 80 µl de pyridine et incubés durant 25 minutes à 80°C avec 80 µl de sylon (BSTFA : TMCS, 99:1, Supelco). Après évaporation douce des réactifs en excès sous jet d'azote, les méthylglycosides triméthylsilylés sont repris dans 500 µl de dichlorométhane puis injectés en chromatographie en phase gazeuse (injection in-colonne, détecteur FID : ionisation de flamme). Le gaz vecteur est l'azote. La colonne, de type HP-5MS (30 m, 0.25 mm de diamètre interne), est apolaire. Le programme de montée en température est le suivant: 120°C maintenus pendant 1 minute, puis un gradient de 1.5°C/min jusqu'à 180°C, suivi d'un gradient de 2°C/min jusqu'à 200°C.

Chaque monosaccharide est identifié par comparaison de ses temps de rétention relatifs par rapport à l'étalon interne, avec ceux des monosaccharides purs traités dans les mêmes conditions. Un coefficient de réponse est calculé pour chaque monosaccharide par rapport à l'étalon interne afin de définir la proportion de chaque monosaccharide au sein des polysaccharides de l'extrait d'algues

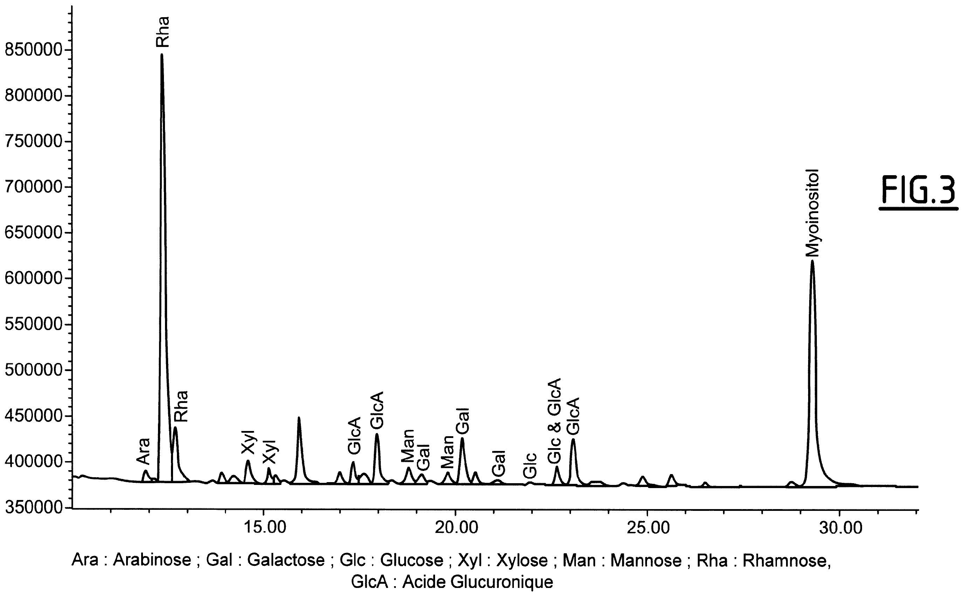

Les résultats obtenus sont présentés dans le tableau 1 ci-dessous et en figure 3.

**Tableau 1 : Composition de l'extrait d'algues obtenue après l'analyse de dérivés triméthylsilylés par chromatographie en phase gazeuse, exprimée en poids par rapport au poids total de l'extrait d'algues; avec Ara: Arabinose ; Gal: Galactose ; Glc: Glucose ; Xyl: Xylose ; Man: Mannose ; Rha: Rhamnose, GlcA: Acide Glucuronique.**

| Echantillon | % en poids de l'ultrafiltrat | Rendement ultrafiltration | % en poids de l'extrait brut |
|---|---|---|---|
| Ara | 0,6 | 20,50% | 0,123 |
| Gal | 1,3 | 20,50% | 0,267 |
| Glc | 0,1 | 20,50% | 0,021 |
| Xyl | 2,45 | 20,50% | 0,502 |
| Man | 0,45 | 20,50% | 0,092 |
| Rha | 39,6 | 20,50% | 8,118 |
| GlcA | 12,9 | 20,50% | 2,645 |

### Exemple 4 : Evaluation des effets de l'extrait d'algue (EA) au travers d'une batterie fonctionnelle de tests et de tests complémentaires (openfield, labyrinthe en croix surélevé, tests d'évitement d'un stimulus aversif)

Les effets d'un extrait d'algue (EA) administré par voie orale aux doses de 20, 40 et 80 mg/kg pendant 14 jours ont été évalués chez des rats mâles Wistar adultes.

Les effets de l'EA ont été évalués sur des groupes de 6 rats par rapport à un lot témoin traité avec le véhicule (eau de source) au travers d'une batterie fonctionnelle de tests (FOB). Des tests complémentaires, l'openfield, le labyrinthe en croix surélevé et le test d'évitement d'un stimulus lumineux aversif, ont été réalisés pour compléter l'évaluation des effets de l'EA.

Les rats utilisés ont été traités conformément aux règles éthiques édictées par l'ASAB et le Conseil Canadien de Protection des Animaux.

### Matériels et méthodes

### Animaux

Vingt quatre (24) rats mâles Wistar Crl:WI (Han) (Charles River Laboratories, 69-StGermain sur l'Arbresle, France) âgés de 60 jours ont été utilisés. A la réception, les rats ont été marqués et répartis par groupes de 2 dans des cages en polycarbonate de type F (48 x 27 x 20 cm, U.A.R., 91 - Epinay-Sur-Orge, France). Les animaux ont été stabulés dans une animalerie climatisée, à une température de 22 ± 2°C et une hygrométrie de 50 ± 20%. Les rats ont disposé de nourriture standard 2016 (Mucedola pour Harlan, Milan, Italie) et de boisson *ad ibitum* et ont été soumis à un cycle lumière-obscurité inversé de 12 heures (lumière de 20h à 08h).

Après une familiarisation d'une semaine aux conditions du laboratoire, les rats ont été pesés et répartis au hasard en 4 groupes de traitements (n = 6).

Afin d'éviter les interférences éventuelles entre les divers produits, les rats d'une même cage ont tous reçu le même traitement. Les rats des différents groupes ont été tous manipulés de la même façon et dans les mêmes conditions.

### Test de la FOB

Les matériaux suivants ont été utilisés dans ce test :
- une cage d'observation transparente ;
- un dispositif de suspension ;
- un dispositif d'agrippement;
- un openfield pour l'activité locomotrice et exploratoire ;
- un appareil de stimulation auditive émettant un stimulus sonore standard ;
- un stylet pour diverses stimulations tactiles ;
- un thermomètre digital.

Le test de la FOB a été réalisé en aveugle avant l'administration du produit (T0) et après une période de 14 jours de traitement (T14) par voie orale aux doses de 20, 40 et 80 mg/Kg.

Le test a comporté trois phases d'observation :
- une phase d'observation directe durant laquelle l'animal n'était pas dérangé ;
- une phase d'observation active pendant laquelle l'animal était manipulé ;
- une phase consacrée à l'évaluation des réactions comportementales des animaux suite à des tests de réactivité.

Les variables enregistrées étaient les suivantes :
- Effets comportementaux : activité locomotrice spontanée, troubles du comportement locomoteur, réaction de fuite au toucher, irritabilité, comportements d'agressivité et de freezing provoqués, somnolence, miction et défécation, réponses sensorimotrices (placement visuel, pincement de la patte, pincement de la queue et réaction à un stimulus sonore).
- Effets neurologiques : réponse pupillaire, réflexe palpébral, élévation pelvienne, position de la queue, tonicité musculaire des membres et de l'abdomen, test de retournement, test d'agrippement, tremblements et piloérection.
Effets physiologiques: salivation, lacrymation, diarrhée, température rectale, rythme cardiaque-respiratoire. - Mortalité.

Les tests suivants ont été réalisés après les deux semaines d'administration des produits :

### Test du labyrinthe en croix surélevé (LCS)

Le dispositif expérimental, en forme de croix, est surélevé à 80 cm au dessus du sol. Il comprend quatre bras de 50 cm de longueur et de 15 cm de largeur. Deux bras opposés sont fermés par des parois verticales de 40 cm de hauteur, les deux autres étant ouverts sur le vide et, de ce fait, représentent des emplacements anxiogènes. Au jour 18, une heure après l'administration du traitement, l'animal a été placé au centre de la croix du dispositif et a pu accéder librement à l'ensemble des quatre bras. Le comportement de l'animal a été enregistré à l'aide du système ANYMAZE pendant 5 minutes. Les variables étudiées dans ce test étaient le nombre d'entrées dans les bras ouverts, ainsi que le temps passé dans ces bras ouverts. Un faible nombre d'entrées et un temps court passé dans les bras ouverts sont considérés comme des indices d'anxiété (Lister, 1987).

### Test d'évitement d'un stimulus lumineux aversif (TESLA)

Ce modèle utilise l'aversion du rat pour un environnement fortement éclairé. Le rat apprend à maîtriser son environnement lumineux aversif dans le cadre d'un conditionnement opérant en appuyant sur un levier actif pour obtenir des périodes d'obscurité de 30 secondes comme renforcement positif.

Le dispositif expérimental consiste en une cage isolée (50 x 40 x 37 cm), fortement éclairée et comportant deux leviers: l'un actif, permettant, lorsqu'il est actionné, d'obtenir 30 secondes d'obscurité suivies du retour de la lumière, alors que l'autre levier est inactif (n'entraîne pas de renforcement positif). Les appuis sur le levier actif, pendant la période d'obscurité, ne procurent pas de périodes d'obscurité supplémentaires. Le rat est placé dans la cage pendant 20 minutes et le nombre d'appuis sur chaque levier est comptabilisé au cours de l'expérimentation.

La batterie de test, composée de 4 dispositifs de conditionnement, est entièrement automatisée et pilotée par ordinateur. Ainsi, aucun expérimentateur n'est présent dans la pièce pendant le test.

Après 18 jours d'administration des produits à tester, les rats ont été testés au plan cognitif pour l'acquisition d'un apprentissage dans le cadre d'un conditionnement opérant dans le modèle du TESLA.

Les variables enregistrées étaient les nombres totaux d'appuis sur les leviers actif (LA) et inactif (LI) et les nombres des appuis sur chacun des deux leviers pendant la phase de lumière.

Les nombres d'appuis actifs et inactifs ont permis d'évaluer le niveau de l'activité manipulatoire au cours de la session de test. L'acquisition de l'apprentissage (discrimination entre les deux leviers) a été évaluée en comparant les nombres d'appuis sur chacun des deux leviers pendant la phase de lumière (LA vs. LI).

### Produit

Le produit est un EA (Extrait d'algues) sous forme de poudre représentant une fraction concentrée lyophilisée et broyée d'un extrait hydrosoluble d'algues préparé selon l'exemple n°1. L'EA est dissout dans de l'eau de source et administré par voie orale 60 minutes avant le test de la FOB et des tests comportementaux complémentaires à l'aide d'une sonde de gavage intragastrique.

### Analyse statistique

Le test de KrusKal-Wallis a été appliqué, suivi, dans les cas d'hétérogénéité, de comparaisons à l'aide du test U de Mann-Whitney pour comparer les groupes traités au groupe témoin.

Le test de Wilcoxon a été utilisé pour les comparaisons deux à deux des mesures répétées de la FOB, entre T0 et T14 dans chacun des groupes et pour la discrimination entre les deux leviers du TESLA en lumière.

Les traitements statistiques et graphiques ont été réalisés à l'aide du logiciel StatvieW 5 (SAS Institute, Inc., USA).

### Résultats

### Test de la FOB

### Comparaisons des groupes traités au groupe véhicule dans les FOB à T0 et T14

Dans la FOB à T0, avant l'administration du traitement, les résultats de tous les groupes de rats étudiés sont homogènes (Tableau 2, Tableau 3).

Dans les tests de la FOB à T14 (après 14 jours d'administration des traitements), le test de KrusKal-Wallis a montré une homogénéité des résultats au sein de nombreuses variables parmi les différents groupes de traitements (Tableau 2).

En revanche, sur certaines variables étudiées, l'EA, à certaines doses, a montré des effets significatifs comparativement au véhicule (Tableau 3).

Les variables principales modifiées par l'administration quotidienne de l'EA pendant deux semaines sont:
- Le comportement des rats et leur activité locomotrice spontanée en cage d'habitation : les rats traités aux 3 doses sont plus actifs et plus alertes,
- Le comportement des rats en cage d'observation: là également, les rats sont plus actifs et plus alertes aux 2 plus fortes doses,
- Intérêt pour un objet présenté : aux 3 doses, l'EA semble avoir un effet stimulant sur la curiosité des rats et leur intérêt pour la nouveauté (augmentation de l'activité exploratoire, hédonisme, effet antidépresseur),
- Les réactions de fuite des rats à l'approche d'un doigt ou au toucher sont significativement diminuées par l'EA aux 3 doses, ce qui relève d'un effet anti-stress /anxiolytique de l'EA,
- Les réactions au pincement de la queue et au pincement de la patte sont également diminuées de façon significative aux 3 doses. Ces baisses de réaction relève d'un effet anti-stress /anxiolytique de l'EA et/ou d'un effet antalgique,
- Les fréquences cardiaque et respiratoire des rats semblent moins élevées chez les rats traités à l'EA aux 3 doses, relèvent d'un effet anti-stress /anxiolytique de l'EA,
- L'agitation, la nervosité et l'irritabilité des rats ont significativement baissé sous administration de l'EA aux 2 doses les plus fortes, relevant d'un effet calmant, anti-stress et/ou anti-agressivité.

**Tableau 2 : Etat des variables avant (T0) et après (T14) l'administration de l'EA pendant 14 jours aux doses de 20, 40 et 80 mg/kg, PO**

| Session de FOB | T0 | | | | T14 | | | |
|---|---|---|---|---|---|---|---|---|
| Groupes Items | Véhicule | EA 20 | EA 40 | EA 80 | Véhicule | EA 20 | EA 40 | EA 80 |
| Latence de déplacement dans l'arène | H(ddl3) = 5.14; P = 0.16 | | | | H(ddl3) = 4.10; P = 0.25 | | | |
| Activité locomotrice (cases traversées) | H(ddl3) = 3.06; P = 0.38 | | | | H(ddl3) = 2.43; P = 0.49 | | | |
| Activité locomotrice (redressements) | H(ddl3) = 4.91; P = 0.18 | | | | H(ddl3) = 2.96; P = 0.40 | | | |
| Etat d'éveil ou de somnolence | H(ddl3) = 3.00; P = 0.39 | | | | H(ddl3) = 0.00; P = 1.00 | | | |
| Cases traversées sur une branche ouverte | H(ddl3) = 3.00; P = 0.39 | | | | H(ddl3) = 3.94; P = 0.27 | | | |
| Réaction de retournement | H(ddl3) = 0.00; P = 1.00 | | | | H(ddl3) = 0.00; P = 1.00 | | | |
| Réaction d'agrippement | H(ddl3) = 1.11 ; P = 0.77 | | | | H(ddl3) = 2.09; P = 0.55 | | | |
| Test de suspension | H(ddl3) = 3.15; P = 0.37 | | | | H(ddl3) = 1.89; P = 0.59 | | | |
| Réaction de sursaut à un stimulus sonore | H(ddl3) = 4.26; P = 0.23 | | | | H(ddl3) = 2.13; P = 0.55 | | | |
| Agressivité provoquée | H(ddl3) = 0.00; P = 1.00 | | | | H(ddl3) = 0.00; P = 1.00 | | | |
| Tonicité musculaire générale | H(ddl3) = 3.34; P = 0.34 | | | | H(ddl3) = 6.39; P = 0.10 | | | |
| Tonicité musculaire des membres | H(ddl3) = 4.60; P = 0.20 | | | | H(ddl3) = 3.29; P = 0.35 | | | |
| Réaction de placement visuel sur la tige | H(ddl3) = 5.63; P = 0.13 | | | | H(ddl3) = 1.97; P = 0.58 | | | |
| Cris | H(ddl3) = 2.30; P = 0.51 | | | | H(ddl3) = 4.31 ; P = 0.23 | | | |
| Défécation | H(ddl3) = 5.43; P = 0.14 | | | | H(ddl3) = 3.22 ; P = 0.36 | | | |
| Miction | H(ddl3) = 6.27; P = 0.10 | | | | H(ddl3) = 2.30; P = 0.51 | | | |
| Convulsions | H(ddl3) = 0.00; P = 1.00 | | | | H(ddl3) = 0.00; P = 1.00 | | | |
| Tremblements | H(ddl3) = 0.00; P = 1.00 | | | | H(ddl3) = 0.00; P = 1.00 | | | |
| Diarrhée | H(ddl3) = 0.00; P = 1.00 | | | | H(ddl3) = 0.00; P = 1.00 | | | |
| Exophtalmie | H(ddl3) = 0.00; P =1.00 | | | | H(ddl3) = 0.00; P =1.00 | | | |
| Piloérection | H(ddl3) = 0.00; P =1.00 | | | | H(ddl3) = 0.00; P =1.00 | | | |
| Salivation | H(ddl3) = 0.00; P =1.00 | | | | H(ddl3) = 0.00; P =1.00 | | | |
| Lacrymation | H(ddl3) = 0.00; P =1.00 | | | | H(ddl3) = 0.00; P =1.00 | | | |
| Réflexe palpébral | H(ddl3) = 0.00; P =1.00 | | | | H(ddl3) = 0.00; P =1.00 | | | |
| Comportements bizarres | H(ddl3) = 0.00; P =1.00 | | | | H(ddl3) = 0.00; P =1.00 | | | |
| Courbure du tronc | H(ddl3) = 0.00; P =1.00 | | | | H(ddl3) = 0.00; P =1.00 | | | |
| Elévation pelvienne | H(ddl3) = 0.00; P =1.00 | | | | H(ddl3) = 0.00; P =1.00 | | | |
| Elévation de la queue | H(ddl3) = 0.00; P =1.00 | | | | H(ddl3) = 0.00; P =1.00 | | | |
| Température rectale | H(ddl3) = 0.17; P =0.98 | | | | H(ddl3) = 3.67; P =0.30 | | | |
| Mortalité à 24h | H(ddl3) = 0.00; P =1.00 | | | | H(ddl3) = 0.00; P =1.00 | | | |

**Tableau 3 : Etat des variables avant (T0) et après (T14) l'administration de l'EA pendant 14 jours aux doses de 20, 40 et 80 mg/kg, PO**

| Session de FOB | T0 | | | | T14 | | | |
|---|---|---|---|---|---|---|---|---|
| Groupes | Véhicule | EA | EA | EA | Véhicule | EA | EA | EA |
| Items | | 20 | 40 | 80 | | 20 | 40 | 80 |
| Comportement du rat en cage d'habitation | H(ddl3) = 5.15 ; P = 0.16 | | | | H(ddl3) = 19.97; P = 0.0002 (D1 ** - D2** - D3**) | | | |
| Comportement du rat en cage d'observation | H(ddl3) = 4.02 ; P = 0.26 | | | | H(ddl3) = 7.19; P = 0.066 (D2# - D3*) | | | |
| Activité locomotrice spontanée en cage d'habitation | H(ddl3) = 4.33; P = 0.23 | | | | H(ddl3) = 15.20; P = 0.008 (D1 ** - D2** - D3**) | | | |
| Intérêt pour un objet présenté (curiosité) | H(ddl3) = 4.86 ; P = 0.18 | | | | H(ddl3) = 6.86; P = 0.08 (D1* - D2# - D3*) | | | |
| Réaction de fuite à l'approche d'un doigt | H(ddl3) = 2.39 ; P = 0.50 | | | | H(ddl3) = 6.65; P = 0.08 (D1#- D2#- D3*) | | | |
| Réaction au pincement de la queue | H(ddl3) = 5.50 ; P = 0.14 | | | | H(ddl3) = 15.07; P = 0.002 (D1 ** - D2** - D3**) | | | |
| Réaction au pincement d'une patte arrière | H(ddl3) = 4.13 ; P = 0.25 | | | | H(ddl3) = 9.63; P = 0.02 (D1 ** - D2# - D3#) | | | |
| Réaction de fuite au toucher | H(ddl3) = 2.12 ; P = 0.55 | | | | H(ddl3) = 8.01 ; P = 0.045 (D1* - D2# - D3#) | | | |
| Fréquence cardiaque et respiratoire | H(ddl3) = 4.60 ; P = 0.20 | | | | H(ddl3) = 10.78; P = 0.01 (D1# - D2** - D3**) | | | |
| Freezing provoqué | H(ddl3) = 3.00; P = 0.39 | | | | H(ddl3) = 6.27; P = 0.09 | | | |
| Tonicité musculaire de l'abdomen | H(ddl3) = 3.83 ; P = 0.28 | | | | H(ddl3) = 8.52 ; P = 0.04 (D2# - D3*) | | | |
| Agitation-nervosité-irritabilité | H(ddl3) = 1.04 ; P = 0.79 | | | | H(ddl3) = 6.77; P = 0.08 (D2# - D3*) | | | |

| | | | | | | | | |
|---|---|---|---|---|---|---|---|---|
| # P < 0.10 ; * P < 0.05 ; ** P < 0.01 (Test de Mann-Whitney: vs. Véhicule) | | | | | | | | |

### Comparaisons des différentes variables répétées entre les FOB à TO et T14

L'administration quotidienne d'EA à certaines doses, pendant 14 jours, semble avoir un effet sur certaines variables principales étudiées (Tableau 4) :
- Le comportement des rats et leur activité locomotrice spontanée en cage d'observation ont augmenté de façon significative sous EA aux 3 doses (effet stimulant ou effet anti- stress),
- La latence de déplacement des rats témoins dans l'arène d'observation augmente et leur activité locomotrice (cases traversées) diminue, alors que chez les rats traités avec l'EA ces variables restent stables entre les FOB T0 et T14 (effets anti-stress et antidépresseur),
- L'intérêt pour un objet présenté : à la dose de 20 mg/Kg, PO (per os), l'EA stimule l'intérêt des rats pour un objet-stimulus qui lui est présenté (motivation à l'exploration de la nouveauté, hédonisme : effets anti-stress et antidépresseur),
- Le nombre de cases traversées sur une branche ouverte : comparativement au véhicule, l'EA ne diminue pas le nombre de cases traversées sur une branche ouverte (effet anti- stress),
- Les réactions de fuite à l'approche d'un doigt : l'EA, aux 3 doses, diminue la réaction de fuite à l'approche du doigt de l'expérimentateur (effet anti-stress),
- La réaction au pincement de la queue augmente chez le rat témoin, alors que sous 20 mg/Kg d'EA elle diminue (effet anti-stress et/ou effet antalgique),
- La réaction au pincement d'une patte arrière diminue sous l'EA, aux 3 doses (effet anti- stress et/ou effet antalgique),
- La réaction de sursaut à un stimulus sonore diminue significativement aux doses d'EA de 40 et 80 mg/Kg (effets calmant et anti-stress),
- Les fréquences cardiaque et respiratoire diminuent sous EA aux doses de 20 et 40 mg/Kg (effets calmant et anti-stress),
- L'agitation, la nervosité et l'irritabilité : à la forte dose de 80 mg/Kg, l'EA montre un effet positif sur l'agitation, la nervosité et l'irritabilité des rats (effets calmant, anti-stress et/ou anti-agressivité).

**Tableau 4 : Effets de l'EA sur l'évolution des variables entre les FOB à T0 et T14 dans chaque groupe de traitement**

| **Groupes** | **VEH** | | **EA/20** | | **EA/40** | | **EA/80** | |
|---|---|---|---|---|---|---|---|---|
| **Session de FOB Items** | **T0** | **T14** | **T0** | **T14** | **T0** | **T14** | **T10** | **T14** |
| Comportement du rat en cage d'observation | N.S. | | 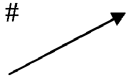 | | 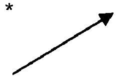 | | 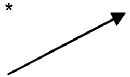 | |
| Activité locomotrice spontanée en cage d'observation | N.S. | | 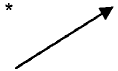 | | 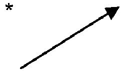 | | 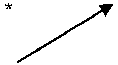 | |
| Latence de déplacement dans l'arène | 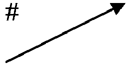 | | N.S. | | N.S. | | N.S. | |
| Activité locomotrice (cases traversées) | 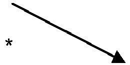 | | N.S. | | N.S. | | N.S. | |
| Intérêt pour un objet présenté | N.S. | | 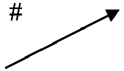 | | N.S. | | N.S. | |
| Cases traversées sur une branche ouverte | 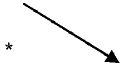 | | N.S. | | N.S. | | N.S. | |
| Réaction de fuite à l'approche d'un doigt | N.S. | | 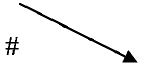 | | 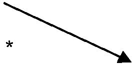 | | 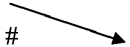 | |
| Réaction au pincement de la queue | 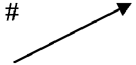 | | 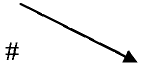 | | N.S. | | N.S. | |
| Réaction au pincement d'une patte arrière | N.S. | | 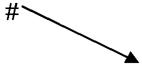 | | 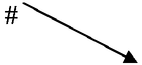 | | 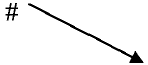 | |
| Test de suspension | 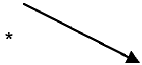 | | N.S. | | 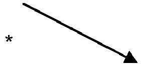 | | 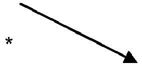 | |
| Réaction de sursaut à un stimulus sonore | N.S. | | N.S. | | 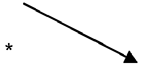 | | 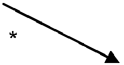 | |
| Fréquence cardiaque et respiratoire | N.S. | | 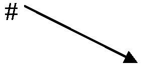 | | 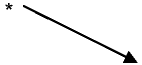 | | N.S. | |
| Tonicité musculaire de l'abdomen | 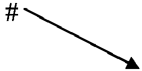 | | N.S. | | N.S. | | N.S. | |
| Agitation-nervosité-irritabilité | N.S. | | N.S. | | N.S. | | 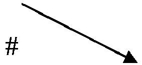 | |

- N.S. :: Non significatif entre T0 et T14
- ↘: Diminue entre T0 et T14
- ↗: Augmente entre T0 et T14
- * :: P < 0.05 ; #: P < 0.10 (tendance)

### Test du labyrinthe en croix surélevé (LCS)

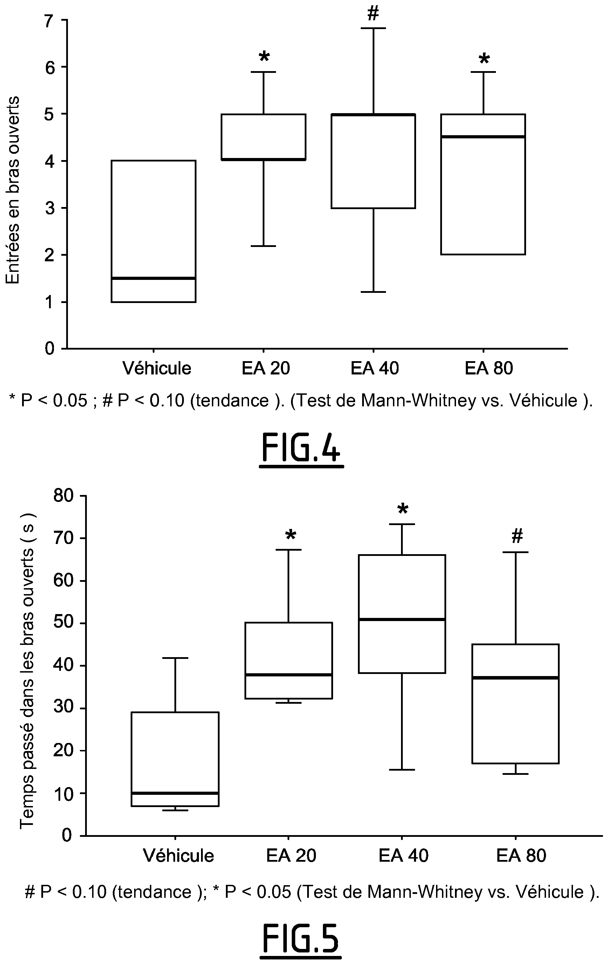

Dans le test du labyrinthe en croix surélevé, après 18 jours de traitement quotidien, l'EA, aux doses de 20, 40 et 80 mg/Kg, PO, augmente significativement le nombre d'entrées (Tableau 5, Figure 4) ainsi que le temps passé (Tableau 6, Figure 5) des rats traités dans les bras ouverts, démontrant une activité anti-stress de l'EA.

**Tableau 5 : Effets de l'EA sur le nombre d'entrées dans les bras ouverts dans le test du labyrinthe en croix surélevé**

| Produits | Véhicule (n = 6) | EA 20 mg/Kg (n = 6) | EA 40 mg/Kg (n = 6) | EA 80 mg/Kg (n = 6) | Test de Kruskal-Wallis |
|---|---|---|---|---|---|
| Nombre d'entrées en branches ouvertes Médiane (QI-QS) | 1.5 (1.0-4.0) | 4.0 (4.0-5.0) | 5.0 (3.0-5.0) | 4.5 (2.0-5.0) | H(ddl3) = 6.08 P = 0.108 |

**Tableau 6 : Effets de l'EA sur le temps passé dans les bras ouverts dans le test du labyrinthe en croix surélevé**

| Produits | Véhicule (n = 6) | EA 20 mg/Kg (n = 6) | EA 40 mg/Kg (n = 6) | EA 80 mg/Kg (n = 6) | Test de Kruskal-Wallis |
|---|---|---|---|---|---|
| Temps passé en branches ouvertes Médiane (QI-QS) | 10.0 (7.0-29.0) | 37.5 (32.0-50.0) | 50.5 (38.0-66.0) | 37.0 (17.0-45.0) | H(ddl3) = 7.76 P = 0.051 |

### Test d'évitement d'un stimulus lumineux aversif (TESLA)

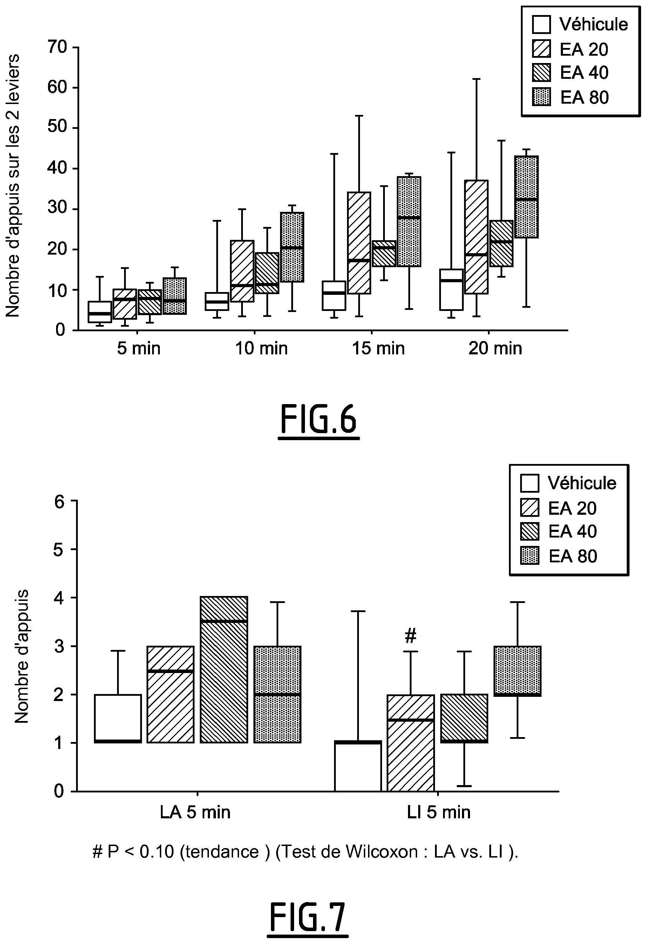

Après 18 jours d'administration quotidienne de l'EA, le test de KrusKal-Wallis a montré que le nombre total d'appuis cumulés sur les deux leviers (levier actif+levier inactif) des rats des différents groupes de traitements sont homogènes à 5 minutes, 10 minutes, 15 minutes et à 20 minutes (Figure 6).

Dans le Test d'évitement d'un stimulus lumineux aversif, à la fin des 5 premières minutes de test, après 18 jours d'administration de l'EA, les rats traités à la dose de 20 mg/Kg, PO, tendent à discriminer le levier actif du levier inactif (Figure 7).

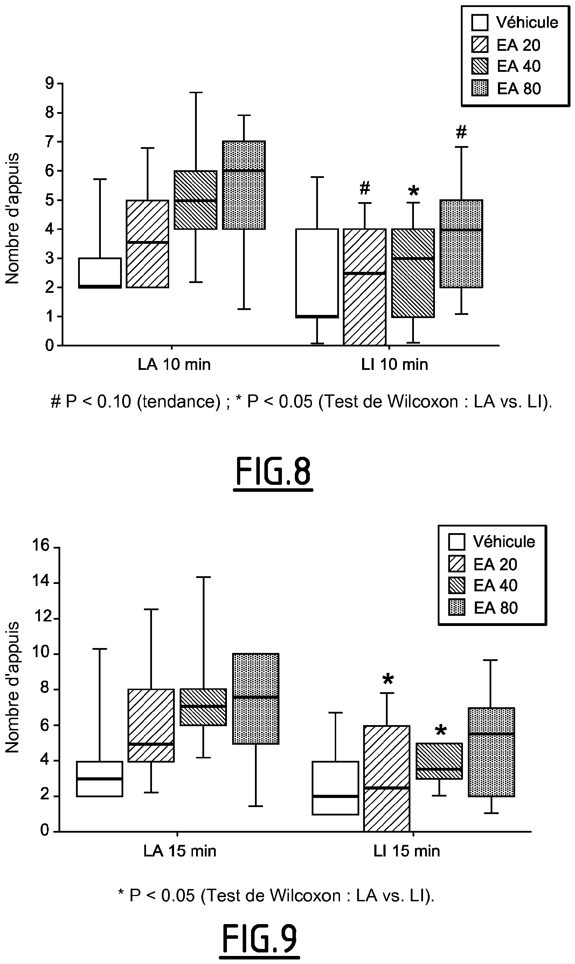

A la fin de la période de 10 minutes de test, les rats traités avec l'EA à la dose de 40 mg/Kg, PO, discriminent significativement le levier actif du levier inactif et ceux traités aux doses de 20 et 80 mg/Kg, PO, tendent à opérer une discrimination entre les deux leviers. Ce n'est pas le cas des rats témoins qui ne montrent aucune tendance à la discrimination (Figure 8).

A la fin de la période de 15 minutes de test, les rats traités avec l'EA aux doses de 20 et 40 mg/Kg, PO, discriminent significativement le levier actif du levier inactif. Les rats traités à la dose de 80 mg/Kg, PO, ne discriminent plus les deux leviers. Les rats témoins ne montrent toujours pas de discrimination à ce stade du test d'apprentissage (Figure 9).

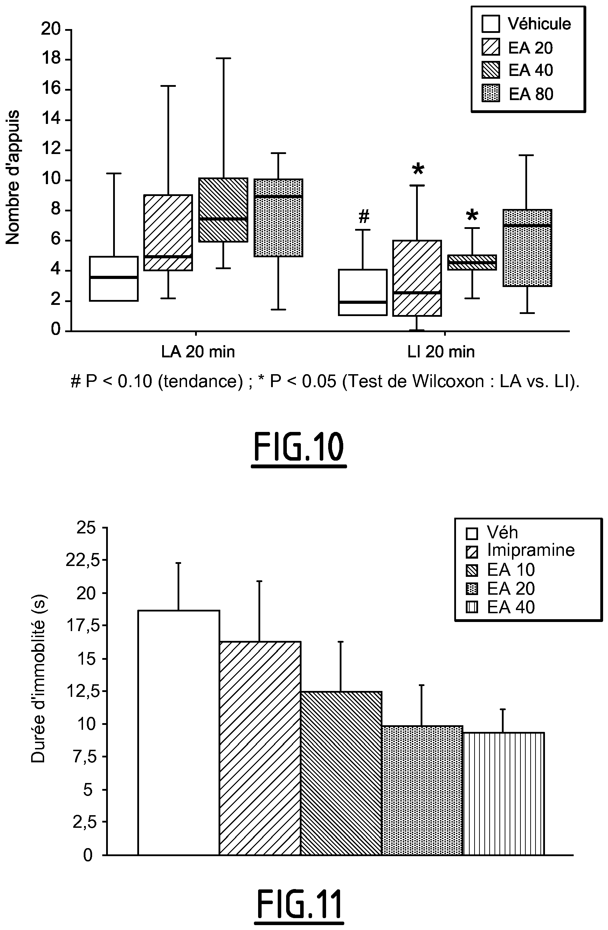

A la fin de la période de 20 minutes de test, les rats traités à l'EA aux doses de 20 et 40 mg/Kg, PO, discriminent toujours significativement le levier actif du levier inactif. Les rats traités à la dose de 80 mg/Kg, PO, ne montrent plus de discrimination et les rats témoins commencent à montrer un début de discrimination (Figure 10).

Ces résultats montrent l'efficacité de l'EA sur l'acquisition d'un apprentissage, et notamment aux doses de 20 et 40 mg/Kg, PO.

### Données physiologiques et d'innocuité

L'administration de l'EA, quelle que soit la dose, n'a pas montré d'influence sur le poids des rats traités quotidiennement aux doses de 20, 40 ou 80 mg/Kg, PO, ni sur leur prise alimentaire. Aucune toxicité n'a été décelée chez les animaux traités à l'EA aux doses de 20, 40 et 80 mg/Kg/j, PO, pendant 21 jours.

### Conclusion

L'administration d'un extrait d'algues selon la présente invention met ainsi en évidence des effets neurologiques du produit, notamment des activités anti-stress/anxiolytique, anti-dépressive, antalgique, ainsi qu'une activité d'acquisition précoce de l'apprentissage et de la facilitation de la mémoire.

### Exemple 5 : Evaluation des effets de l'extrait d'algue (EA) au travers du test de désespoir comportemental

Les effets anti-dépresseurs d'un extrait d'algue selon l'invention (EA), administré par voie orale en traitement préventif à 3 doses (10, 20 et 40 mg/kg/j) ont été évalués chez des rats mâles Wistar adultes dans le test du désespoir comportemental (TDC).

L'extrait a été administré quotidiennement en traitement semi-chronique pendant 14 jours. Les effets de l'extrait ont été évalués dans le TDC sur des groupes de 12 rats par rapport à un lot témoin traité avec le véhicule (eau de source) et un lot Référence traité avec de l'Imipramine à la dose de 10mg/kg/j.

Les rats utilisés ont été traités conformément aux règles éthiques édictées par l'ASAB et le Conseil Canadien de Protection des Animaux.

### Matériels et méthodes

### Animaux

Soixante rats mâles Wistar (Charles River Laboratories, France) pesant 200-225 g ont été utilisés. A la réception les rats ont été marqués et répartis par groupes de 4 dans des cages en polycarbonate de type F (48 x 27 x 20 cm, U.A.R., 91 - Epinay-Sur-Orge, France). Les animaux ont été stabulés dans une animalerie climatisée, à une température de 22 ± 2°C et une humidité relative de 50 ± 20 %. Les rats ont disposé de nourriture standard 2016 (Harlan, Gannat, France) et de boisson *ad libitum.* Ils ont été soumis à un cycle lumière-obscurité inversé de 12 heures.

Après une familiarisation d'une semaine aux conditions du laboratoire, les rats ont été pesés et randomisés en fonction de leur poids en 5 groupes de traitements (n = 12/groupe).

Groupes de traitement :
- Groupe Témoin : traitement avec de l'eau de source (Véh),
- Groupe Imipramine : traitement avec de l'Imipramine à la dose de 10 mg/kg/j (Imi),
- Groupe EA 10 : traitement avec l'EA à la dose de 10 mg/kg/j (EA 10),
- Groupe EA 20 : traitement avec l'EA à la dose de 20 mg/kg/j (EA 20),
- Groupe EA 40 : traitement avec l'EA à la dose de 40 mg/kg/j (EA 40).

Afin d'éviter les interférences éventuelles entre les différents traitements, les rats d'une même cage ont tous reçu la même dose d'EA. Les rats des différents groupes ont tous été manipulés de la même façon et dans les mêmes conditions.

### Test du désespoir comportemental (TDC)

Les dispositifs expérimentaux consistaient en des cylindres en Plexiglas de 50 cm de hauteur et de 20 de diamètre remplis d'eau à 25°C jusqu'à une hauteur de 30 cm.

Après 13 jours de traitement quotidien avec les produits à tester, les rats ont été placés à J14, dans les dispositifs pendant une durée de 15 minutes pour évaluer le temps d'immobilité de chaque animal. Immédiatement après ce premier test, les rats ont reçu leur 14^{ème} traitement.

A J15, les rats ont reçu deux traitements : le premier, 5 heures avant le test et le second 1 heure avant le test. Cette procédure est usuellement utilisée dans la littérature pour tester l'efficacité d'un produit contre la dépression. Le test a été réalisé dans les mêmes conditions que la veille pendant une durée de 5 minutes.

La durée d'immobilité enregistrée pendant les 5 premières minutes des deux tests (à J14 et J15) reflète la capacité de l'animal à se résigner lorsqu'il est confronté à une situation d'où il ne peut s'échapper. Ce comportement de résignation est assimilé à de la dépression.

### Produit

Le produit est un EA (Extrait d'algues) sous forme de poudre représentant une fraction concentrée lyophilisée et broyée d'un extrait hydrosoluble d'algues préparé selon l'exemple n°1. L'extrait à tester a été dissout dans de l'eau de source et a été administré aux rats par voie orale à l'une des 3 doses testées pendant 14 jours successifs avec un volume d'administration de 5 ml/kg. Les animaux ont été pesés tous les deux ou trois jours afin d'adapter leurs traitements spécifiques à leurs poids. Leurs prises alimentaire et hydrique ont été relevées tous les deux ou trois jours pendant toute la durée de l'étude.

Les animaux du groupe Référence ont reçu quotidiennement 10 mg/kg/j d'Imipramine hydrochloride (Sigma Aldrich) dissoute dans de l'eau de source avec un volume d'administration de 5 ml/kg.

Les animaux du groupe Véhicule ont reçu quotidiennement de l'eau de source avec un volume d'administration de 5 ml/kg.

### Analyse statistique

Une ANOVA en mode paramétrique a été appliquée, suivie, dans le cas d'hétérogénéité, de comparaisons à l'aide du test-t non apparié pour comparer les groupes traités au groupe Véhicule et au groupe Référence.

Une ANOVA appariée a été utilisée, suivie, dans le cas d'une hétérogénéité, de comparaisons à l'aide du test-t apparié pour les comparaisons deux à deux des mesures répétées du TDC dans chacun des groupes.

Pour les prises alimentaire et hydrique, une analyse en mode non paramètrique a été appliquée : un test de Kruskal-Wallis suivi, le cas échéant par un test U de Mann Whitney pour comparer les groupes deux à deux.

Les traitements statistiques et graphiques ont été réalisés à l'aide du logiciel Statview5 (SAS Institute, Inc., USA).

### Résultats

### Test de désespoir comportemental : Prétest à J14

A J14, dans le prétest du désespoir comportemental, l'ANOVA n'a pas montré d'hétérogénéité pour le temps d'immobilité des animaux des différents groupes de traitement (Tableau 7 ; Figure 11). Ce prétest permet d'obtenir l'induction de la résignation chez les animaux des différents groupes de traitement.

**Tableau 7 : Effets de l'EA sur la durée d'immobilité (s) dans le prétest du désespoir comportemental à J14 (s, Moyenne ± ESM)**

| Produits | **Véhicule** | **Imipramine** | **EA** | **EA** | **EA** | **ANOVA_{(ddl=4)}** |
|---|---|---|---|---|---|---|
| | (n = 12) | 10 mg/kg (n = 12) | 10 mg/kg (n = 12) | 20 mg/kg (n = 12) | 40 mg/kg (n = 12) | |
| **Durée d'immobilité (s)** | 18.7 ± 3.6 | 16.3 ± 4.6 | 12.5 ± 3.8 | 9.8 ± 3.2 | 9.3 ± 1.8 | **F = 1.33** |
| | | | | | | **P = NS** |

| | | | | | | |
|---|---|---|---|---|---|---|
| NS : non significatif. | | | | | | |

### Test de désespoir comportemental à J15

A J15, dans le test du désespoir comportemental, l'ANOVA a montré une hétérogénéité pour le temps d'immobilité des animaux des différents groupes de traitement (Tableau 8).

Le test-t non apparié a montré que les temps d'immobilité des animaux traités avec l'Imipramine, EA 20 et EA 40 étaient significativement plus courts que celui des animaux traités avec le Véhicule (t=3.69, P=0.0013 ; t=2.20, P=0.039 et t=3.29, P=0.003, respectivement).

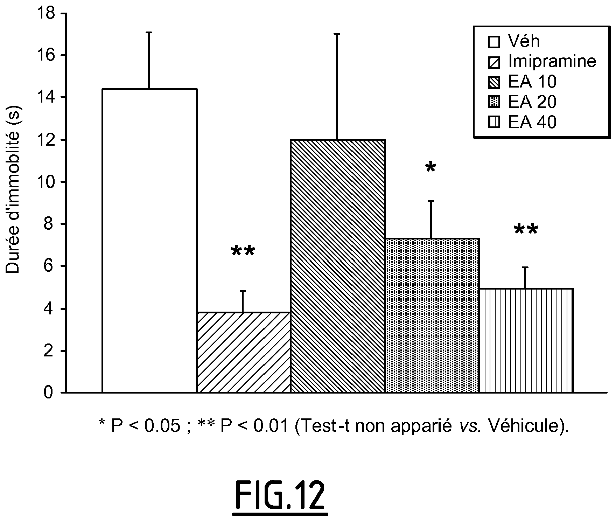

Le test-t non apparié a montré que le temps d'immobilité des animaux traités avec EA 20 tendait à être significativement plus long que celui des animaux traités avec l'Imipramine (t=1.72, P=0.099) (Tableau 8 ; Figure 12).

**Tableau 8 : Effets de l'EA sur la durée d'immobilité (s) dans le test de désespoir comportemental à J15 (s, Moyenne ± ESM)**

| **Produits** | | **Véhicule** | **Imipramine** | | **EA** | | **EA** | | **EA** | | **ANOVA_{(ddl=4)}** | |
|---|---|---|---|---|---|---|---|---|---|---|---|---|
| | | (n = 12) | 10 mg/kg (n = 12) | | 10 mg/kg (n = 12) | | 20 mg/kg (n = 12) | | 40 mg/kg (n = 12) | | | |
| **Durée d'immobilité (s)** | | 14.4 ± 2.7 | 3.8 ± 1.0 | | 12.0 ± 5.0 | | 7.3 ± 1.8 | | 4.9 ± 1.0 | | **F = 2.76** | |
| | | | | | | | | | | | **P** = **0.036** | |
| | **Test-t non apparié** | | | **t = 3.69** | | **t = 0.42** | | **t = 2.20** | | **t = 3.29** | | |
| | **(vs. Véhicule)** | | | **P = 0.0013** | | **NS** | | **P = 0.039** | | **P = 0.003** | | |
| | **Test-t non apparié** | | | | | t = 1.60 | | t = 1.72 | | t = 0.75 | | |
| | **(vs. Imipramine)** | | | | | P = NS | | T (P = 0.099) | | NS | | |
| | **Test-t non apparié** | | | | | | | t = 0.98 | | **t** = **1.38** | | |
| | **(vs. EA 10)** | | | | | | | *NS* | | NS | | |
| | **Test-t non apparié** | | | | | | | | | t = 1.17 | | |
| | **(vs. EA 20)** | | | | | | | | | NS | | |

| | | | | | | | | | | | | |
|---|---|---|---|---|---|---|---|---|---|---|---|---|
| NS : non significatif. | | | | | | | | | | | | |

### Comparaison des résultats des tests à J14 et J15

Le test-t apparié a montré que les durées d'immobilité des rats traités avec l'Imipramine et EA 40 ont significativement diminué entre J14 et J15 (t=2.90, P=0.015 et t=2.42, P=0.034, respectivement) (Tableau 9).

**Tableau 9 : Comparaison des résultats des tests à J14 et à J15 (s, Moyenne ± ESM)**

| **Produits** | **Véhicule** | **Imipramine** | **EA** | **EA** | **EA** |
|---|---|---|---|---|---|
| | (n = 12) | 10 mg/kg (n = 12) | 10 mg/kg (n = 12) | 20 mg/kg (n = 12) | 40 mg/kg (n = 12) |
| **Durée d'immobilité (s)** | 18.7 ± 3.6 | 16.3 ± 4.6 | 12.5 ± 3.8 | 9.8 ± 3.2 | 9.3 ± 1.8 |
| A J14 | | | | | |
| **Durée d'immobilité (s)** | 14.4 ± 2.7 | 3.8 ± 1.0 | 12.0 ± 5.0 | 7.3 ± 1.8 | 4.9 ± 1.0 |
| A J15 | | | | | |
| **Test-t apparié** | | | | | |
| **t=** | 1.41 | **2.90** | 0.11 | 1.10 | **2.42** |
| **P=** | NS | **0.015** | NS | NS | **0.034** |

| | | | | | |
|---|---|---|---|---|---|
| NS : non significatif. | | | | | |

### Evolution pondérale, prises alimentaire et hydrique

Aucun effet de l'EA administré aux trois doses n'a été mis en évidence en ce qui concerne l'évolution pondérale et les prises alimentaire et hydrique des animaux.

### Test de désespoir comportemental

**A J14** : lors du test de mise en place de la résignation chez les animaux, bien que la durée d'immobilité des animaux traités avec l'EA aux doses de 20 et 40 mg/kg soient plus courtes que celles des animaux des autres groupes, aucune différence significative n'a été observée entre les rats traités avec le Véhicule, l'Imipramine et l'EA administré aux trois doses testées.

**A J15** : l'effet anti-dépresseur de l'EA administré aux doses de 20 et 40 mg/kg a été mis en évidence car les durées d'immobilité des animaux traités à ces doses étaient significativement plus courtes que celle des animaux traités avec le Véhicule. L'EA administré à la dose de 40mg/kg a permis d'obtenir une durée d'immobilité similaire à celle des animaux traités avec l'Imipramine à 10mg/kg. Un effet dose-dépendant a été observé chez les animaux traités avec l'EA aux doses de 10, 20 et 40 mg/kg.

**Comparaison des durées d'immobilité à J14 et J15** : seuls les animaux traités avec l'Imipramine à la dose de 10 mg/kg et avec l'EA à la dose de 40 mg/kg ont diminué significativement leurs durées d'immobilité entre J14 et J15.

### Comportement des animaux et innocuité de l'extrait d'algues

Aucun comportement anormal de l'ensemble des animaux n'a été observé au cours de l'expérimentation pour l'EA administré aux doses de 10, 20 et 40 mg/kg. Aucune toxicité n'a été décelée chez les animaux traités avec l'EA aux doses de 10, 20 et 40 mg/kg/j pendant 14 jours.

### Conclusion

L'administration d'un extrait d'algues selon la présente invention met ainsi en évidence un effet anti-dépresseur du produit.

## Revendications

1. Extrait d'algues de l'ordre des ulvales, en particulier extrait d'algues vertes de type *Ulva,* comprenant des polysaccharides polyanioniques sulfatés et non sulfatés dont la taille est inférieure ou égale à 50 kDa, pour utilisation dans une méthode permettant de prévenir et/ou traiter les désordres neurologiques, la dépression, l'anxiété, les douleurs chroniques neuropathiques et inflammatoires, la douleur cutanée (de la peau, des tissus sous-cutanés et organes associés) ou somatique.

2. Extrait d'algues pour son utilisation selon la revendication 1 dans une méthode permettant de prévenir et/ou traiter la dépression, l'anxiété, la maladie d'Alzheimer, les douleurs chroniques neuropathiques et inflammatoires, la douleur cutanée ou somatique.

3. Extrait d'algues pour utilisation selon la revendication 1 ou 2, dans lequel lesdits polysaccharides comprennent du mannose et/ou de l'arabinose.

4. Extrait d'algues pour utilisation selon la revendication 3, dans lequel lesdits polysaccharides comprennent au moins au 0.005% de mannose et/ou au moins 0.005% d'arabinose, en poids par rapport au poids de la matière sèche totale de l'extrait d'algues.

5. Extrait d'algues pour utilisation selon la revendication 4, dans laquelle lesdits polysaccharides comprennent du mannose en une quantité allant de 0.01 à 0.20% et de l'arabinose en une quantité allant de 0.01 à 0.5%, en poids par rapport au poids de la matière sèche totale de l'extrait d'algues.

6. Extrait d'algues pour utilisation selon l'une quelconque des revendications 1 à 4, dans laquelle lesdits polysaccharides comprennent en outre:
- du galactose;
- du glucose;
- du rhamnose;
- du xylose; et
- de l'acide glucuronique.

7. Extrait d'algues pour utilisation selon la revendication 6, dans laquelle lesdits polysaccharides comprennent :
- de 0.05 à 0.5% de galactose;
- de 0.005 à 0.05% de glucose;
- de 2 à 15 % de rhamnose;
- de 0.1 à 1% de xylose; et
- de 1 à 7% d'acide glucuronique;
en poids par rapport au poids de la matière sèche totale de l'extrait d'algues.

8. Extrait d'algues pour utilisation selon l'une quelconque des revendications précédentes, dans lequel lesdits polysaccharides ont une taille inférieure ou égale à 15kDa.

9. Extrait d'algues pour utilisation selon l'une quelconque des revendications précédentes, comprenant :
- de 10 à 50 % de carbone ;
- de 1 à 10% d'hydrogène ;
- de 1 à 5 % d'azote ;
- de 20 à 50 % d'oxygène ; et
- de 1 à 15 % de soufre ;
en pourcentage massique de la matière sèche totale de l'extrait d'algues.

10. Extrait d'algues pour utilisation selon l'une quelconque des revendications précédentes, comprenant :
- de 15 à 30 % de carbone ;
- de 3 à 6% d'hydrogène ;
- de 1 à 3 % d'azote ;
- de 25 à 40 % d'oxygène ; et
- de 2,5 à 10 % de soufre ;
en pourcentage massique de la matière sèche totale de l'extrait d'algues.

11. Extrait d'algues pour utilisation selon l'une quelconque des revendications précédentes, **caractérisé par** le spectre RMN ¹H en Figure 1.

12. Extrait d'algues de l'ordre des ulvales, en particulier extrait d'algues vertes de type *Ulva,* susceptible d'être obtenu par le procédé dans lequel:
a) les algues sont lavées et dessablées ;
b) lesdites algues sont broyées ;
c) la phase solide du broyat est séparée de sa phase liquide ;
d) ladite phase liquide est clarifiée ;
e) le jus obtenu à l'étape d) est ultrafiltré sur une membrane de moins de 50 kDa ; et
f) le jus de filtration obtenu à l'étape e) est concentré puis séché ;
pour utilisation dans une méthode permettant de prévenir et/ou traiter les désordres neurologiques, la dépression, l'anxiété, les douleurs chroniques neuropathiques et inflammatoires, la douleur cutanée (de la peau, des tissus sous-cutanés et organes associés) ou somatique.

13. Extrait d'algues pour son utilisation selon la revendication 12 dans une méthode permettant de prévenir et/ou traiter la dépression, l'anxiété, la maladie d'Alzheimer, les douleurs chroniques neuropathiques et inflammatoires, la douleur cutanée ou somatique.

14. Extrait d'algues pour utilisation selon la revendication 12 ou 13, dans lequel le jus obtenu à l'étape d) dudit procédé est ultrafiltré sur une membrane de 15 kDa.

15. Extrait d'algues pour utilisation selon l'une des revendications 12 à 14, dans lequel l'étape c) dudit procédé est réalisée par pressage du broyat obtenu à l'étape b).

16. Extrait d'algues pour utilisation selon l'une quelconques des revendications 12 à 15, susceptible d'être obtenu par le procédé dans lequel :
a) les algues sont lavées à l'eau douce et dessablées ; et/ou
b) lesdites algues sont broyées avec un affineur ; et/ou
c) la phase solide du broyat est séparée de sa phase liquide par pressage du broyat avec une presse à bande ; et/ou
d) ladite phase liquide est clarifiée avec un clarificateur à assiettes; et/ou
e) le jus obtenu à l'étape d) est ultrafiltré sur une membrane céramique de 15 kDa ; et/ou
f) le jus de filtration obtenu à l'étape e) est concentré par évaporation puis séché par lyophilisation ou atomisation.

17. Composition pharmaceutique comprenant un extrait d'algues tel que défini dans l'une quelconque des revendications 1 à 16 ainsi qu'au moins un excipient pharmaceutiquement acceptable, pour utilisation dans une méthode permettant de prévenir et/ou traiter les désordres neurologiques, la dépression, l'anxiété, les douleurs chroniques neuropathiques et inflammatoires, la douleur cutanée (de la peau, des tissus sous-cutanés et organes associés) ou somatique.

18. Composition pharmaceutique pour son utilisation selon la revendication 17 dans une méthode permettant de prévenir et/ou traiter la dépression, l'anxiété, la maladie d'Alzheimer, les douleurs chroniques neuropathiques et inflammatoires, la douleur cutanée ou somatique.

## Patentansprüche

1. Extrakt von Algen der Ordnung der Ulvales, insbesondere ein Extrakt von Grünalgen des Typs *Ulva* umfassend sulfatierte und nicht-sulfatierte, polyanionische Polysaccharide mit einer Größe von kleiner als oder gleich 50 kDa, zur Verwendung in einem Verfahren, welches die Prävention und/oder die Behandlung neurologischer Erkrankungen, von Depression, Angst, neuropathischen und entzündlichen, chronischen Schmerzen, dermalem (der Haut, von subkutanen Geweben und assoziierten Organen) oder somatischem Schmerz ermöglicht.

2. Extrakt von Algen zur Verwendung gemäß Anspruch 1 in einem Verfahren, welches die Prävention und/oder Behandlung von Depression, Angst, der Alzheimer-Krankheit, von neuropathischen oder entzündlichen, chronischen Schmerzen, dermalem und somatischem Schmerz ermöglicht.

3. Extrakt von Algen zur Verwendung gemäß Anspruch 1 oder 2, wobei die Polysaccharide Mannose und/oder Arabinose umfassen.

4. Extrakt von Algen zur Verwendung gemäß Anspruch 3, wobei die Polysaccharide mindestens 0,005 Gew.-% Mannose und/oder mindestens 0,005 Gew.-% Arabinose umfassen, bezogen auf das Gesamttrockengewicht des Algenextrakts.

5. Extrakt von Algen zur Verwendung gemäß Anspruch 4, wobei die Polysaccharide Mannose in einer Menge von 0,01 bis 0,20 Gew.-% und Arabinose in einer Menge von 0,01 bis 0,5 Gew.-% umfassen, bezogen auf das Gesamttrockengewicht des Algenextrakts.

6. Extrakt von Algen zur Verwendung gemäß einem der Ansprüche 1 bis 4, wobei die Polysaccharide des Weiteren umfassen:
- Galactose;
- Glucose;
- Rhamnose;
- Xylose; und
- Glucuronsäure.

7. Extrakt von Algen zur Verwendung gemäß Anspruch 6, wobei die Polysaccharide umfassen:
- von 0,05 bis 0,5 % Galactose;
- von 0,005 bis 0,05 % Glucose;
- von 2 bis 15 % Rhamnose;
- von 0,1 bis 1 % Xylose; und
- von 1 bis 7 % Glucuronsäure;
bezogen auf das Gesamttrockengewicht des Algenextrakts.

8. Extrakt von Algen zur Verwendung gemäß einem der voranstehenden Ansprüche, wobei die Polysaccharide eine Größe von kleiner oder gleich 15 kDa haben.

9. Extrakt von Algen zur Verwendung gemäß einem der voranstehenden Ansprüche, umfassend:
- von 10 bis 50 % Kohlenstoff;
- von 1 bis 10 % Wasserstoff;
- von 1 bis 5 % Stickstoff;
- von 20 bis 50 % Sauerstoff; und
- von 1 bis 15 % Schwefel;
in Massenprozent des Gesamttrockengewichts des Algenextrakts.

10. Extrakt von Algen zur Verwendung gemäß einem der voranstehenden Ansprüche, umfassend:
- von 15 bis 30 % Kohlenstoff;
- von 3 bis 6 % Wasserstoff;
- von 1 bis 3 % Stickstoff;
- von 25 bis 40 % Sauerstoff; und
- von 2,5 bis 10 % Schwefel;
in Massenprozent des Gesamttrockengewichts des Algenextrakts.

11. Extrakt von Algen zur Verwendung gemäß einem der voranstehenden Ansprüche, **gekennzeichnet durch** das 1H-NMR Spektrum in Abbildung 1.

12. Extrakt von Algen der Ordnung der Ulvales, insbesondere ein Extrakt von Grünalgen des Typs *Ulva*, erhältlich durch das Verfahren, in welchem:
a) die Algen gewaschen und entsandet werden;
b) die Algen zerkleinert werden;
c) die feste Phase des Mahlguts von seiner flüssigen Phase getrennt wird;
d) die flüssige Phase geklärt wird;
e) der in Schritt d) erhaltene Saft auf einer Membran von mindestens 50 kDa ultrafiltriert wird;
f) der in Schritt e) erhaltene Filtrationssaft bis zur Trocknung konzentriert wird;
zur Verwendung in einem Verfahren, welches die Prävention und/oder die Behandlung neurologischer Erkrankungen, von Depression, Angst, neuropathischen und entzündlichen, chronischen Schmerzen, dermalem (der Haut, von subkutanen Geweben und assoziierten Organen) oder somatischem Schmerz ermöglicht.

13. Extrakt von Algen zur Verwendung gemäß Anspruch 12 in einem Verfahren, welches die Prävention und/oder die Behandlung von Depression, Angst, der Alzheimer-Krankheit, neuropathischen und entzündlichen, chronischen Schmerzen, dermalem und somatischem Schmerz ermöglicht.

14. Extrakt von Algen zur Verwendung gemäß Anspruch 12 oder 13, wobei der in Schritt d) des Verfahrens erhaltene Saft auf einer Membran von 15 kDa ultrafiltriert wird.

15. Extrakt von Algen zur Verwendung gemäß einem der Ansprüche 12 bis 14, wobei Schritt c) des Verfahrens mittels Pressung des in Schritt b) erhaltenen Mahlguts durchgeführt wird.

16. Extrakt von Algen zur Verwendung gemäß einem der Ansprüche 12 bis 15, erhältlich durch ein Verfahren, in welchem:
a) die Algen mit Süßwasser gewaschen und entsandet werden; und/oder
b) die Algen mit einem Refiner zerkleinert werden; und/oder
c) die feste Phase des Mahlguts von seiner flüssigen Phase mittels Pressen des Mahlguts mit einer Bandpresse getrennt wird; und/oder
d) die flüssige Phase mit einem Tellerklärbecken geklärt wird; und/oder
e) der in Schritt d) erhaltene Saft auf einer Keramikmembran von 15 kDa ultrafiltriert wird; und/oder
f) der in Schritt e) erhaltene Filtrationssaft durch Verdampfung mittels Lyophilisation oder Atomisierung bis zur Trocknung konzentriert wird.

17. Pharmazeutische Zusammensetzung umfassend ein Algenextrakt wie in einem der Ansprüche 1 bis 16 definiert sowie mindestens einen pharmazeutisch annehmbaren Hilfsstoff, zur Verwendung in einem Verfahren, welches die Prävention und/oder die Behandlung neurologischer Erkrankungen, von Depression, Angst, neuropathischen und entzündlichen, chronischen Schmerzen, dermalem (der Haut, von subkutanen Geweben und assoziierten Organen) oder somatischem Schmerz ermöglicht.

18. Pharmazeutische Zusammensetzung zur Verwendung gemäß Anspruch 17 in einem Verfahren, welches die Prävention und/oder die Behandlung von Depression, Angst, der Alzheimer-Krankheit, neuropathischen und entzündlichen, chronischen Schmerzen, dermalem und somatischem Schmerz ermöglicht.

## Claims

1. An extract of algae from the order of *Ulvales*, in particular an extract of green algae of the type *Ulva,* comprising sulphated and non-sulphated polyanionic polysaccharides, the size of which is less than or equal to 50 kDa, for use in a method for preventing and/or treating neurological disorders, depression, anxiety, neuropathic and inflammatory chronic pain, skin pain (of the skin, of subcutaneous tissues and associated organs) or body pain.

2. The algal extract for use according to claim 1 in a method for preventing and/or treating depression, anxiety, Alzheimer's disease, neuropathic and inflammatory chronic pain, skin pain or body pain.

3. The algal extract for use according to claim 1 or 2, wherein said polysaccharides comprise mannose and/or arabinose.

4. The algal extract for use according to claim 3, wherein said polysaccharides comprise at least 0.005% of mannose and/or at least 0.005% of arabinose, by weight based on the weight of the total dry material of the algal extract.

5. The algal extract for use according to claim 4, wherein said polysaccharides comprise mannose in an amount ranging from 0.01 to 0.20% and arabinose in an amount ranging from 0.01 to 0.5%, by weight based on the weight of the total dry material of the algal extract.

6. The algal extract for use according to any of claims 1 to 4, wherein said polysaccharides further comprise:
- galactose;
- glucose;
- rhamnose;
- xylose; and
- glucuronic acid.

7. The algal extract for use according to claim 6, wherein said polysaccharides comprise:
- from 0.05 to 0.5% of galactose;
- from 0.005 to 0.05% of glucose;
- from 2 to 15% of rhamnose;
- from 0.1 to 1% of xylose; and
- from 1 to 7% of glucuronic acid;
by weight based on the weight of the total dry material of the algal extract.

8. The algal extract for use according to any of the preceding claims, wherein said polysaccharides have a size of less than or equal to 15kDa.

9. The algal extract for use according to any of the preceding claims, comprising:
- from 10 to 50% of carbon;
- from 1 to 10% of hydrogen;
- from 1 to 5% of nitrogen;
- from 20 to 50% of oxygen; and
- from 1 to 15% of sulphur;
in weight percentage of the total dry material of the algal extract.

10. The algal extract for use according to any of the preceding claims, comprising::
- from 15 to 30% of carbon;
- from 3 to 6% of hydrogen;
- from 1 to 3% of nitrogen;
- from 25 to 40% of oxygen; and
- from 2.5 to 10% of sulphur;
in weight percentage of the total dry material of the algal extract.

11. The algal extract for use according to any of the preceding claims, **characterized by** the ¹H NMR spectrum in Fig. 1.

12. An extract of algae from the order of *Ulvales*, in particular an extract of green algae of the type *Ulva,* which may be obtained by a method wherein:
a) the algae are washed and freed of sand;
b) said algae are crushed;
c) the solid phase of the crushed product is separated from its liquid phase;
d) said liquid phase is clarified;
e) the juice obtained in step d) is ultrafiltrated on a membrane of less than 50 kDa; and
f) the filtration juice obtained in step e) is concentrated and then dried;
for use in a method for preventing and/or treating neurological disorders, depression, anxiety, neuropathic and inflammatory chronic pain, skin pain (of the skin, of subcutaneous tissues and associated organs) or body pain.

13. The algal extract for use according to claim 12 in a method for preventing and/or treating depression, anxiety, Alzheimer's disease, neuropathic and inflammatory chronic pain, skin pain or body pain.

14. The algal extract for use according to claim 12 or 13, wherein the juice obtained in step d) is ultrafiltrated on a 15 kDa membrane.

15. The algal extract for use according to one of claims 12 to 14, wherein step c) of said method is carried out by pressing the crushed product obtained in step b).

16. The algal extract for use according to any of claims 12 to 15, which may be obtained by a method wherein:
a) the algae are washed in freshwater and freed of sand; and/or
b) said algae are crushed with a refiner; and/or
c) the solid phase of the crushed product is separated from its liquid phase by pressing the crushed product with a belt press; and/or
d) said liquid phase is clarified with a clarifier with disc stacks; and/or
e) the juice obtained in step d) is ultrafiltrated on a 15 kDa ceramic membrane; and/or
f) the filtration juice obtained in step e) is concentrated by evaporation and then dried by freeze-drying or atomization.

17. A pharmaceutical composition comprising an algal extract according to any of claims 1 to 16 as well as at least one pharmaceutically acceptable excipient, for use in a method for preventing and/or treating neurological disorders, depression, anxiety, neuropathic and inflammatory chronic pain, skin pain (of the skin, of subcutaneous tissues and associated organs) or body pain.

18. A pharmaceutical composition for use according to claim 17 for preventing and/or treating depression, anxiety, Alzheimer's disease, neuropathic and inflammatory chronic pain, skin pain or body pain.
